# EUROPEAN PATENT SPECIFICATION

(11) **EP 4 491 960 B1**
(45) Date of publication and mention of the grant of the patent: **18.02.2026**
(21) Application number: 24181405.2
(22) Date of filing: 11.06.2024
(51) Int. Cl.: F24F 8/10, F24F 11/56, F24F 11/63, F24F 11/89, F24F 110/50, F24F 110/52

(54) **INDOOR AIR POLLUTION PREVENTION SYSTEM**
SYSTEM ZUR VERHINDERUNG VON INNENRAUMLUFTVERSCHMUTZUNG
SYSTÈME DE PRÉVENTION DE POLLUTION D'AIR INTÉRIEUR

(30) Priority: 11.07.2023 TW 112125910
(43) Date of publication of application: 15.01.2025
(73) Proprietor: Microjet Technology Co., Ltd., Hsinchu (TW)
(72) Inventor: Mou, Hao-Jan, Hsinchu (TW); Wu, Chin-Chuan, Hsinchu (TW); Huang, Chi-Feng, Hsinchu (TW)
(74) Representative: Uexküll & Stolberg

(56) References cited:
- WO-A1-2022/266451
- TW-B- I 796 113
- US-B2- 11 480 351
- US-B2- 11 635 221

## Description

### FIELD OF THE INVENTION

The present invention relates to an indoor air pollution prevention system, and more particularly to an indoor air pollution prevention system suitable for various indoor fields.

### BACKGROUND OF THE INVENTION

Suspension particles are solid particles or droplets within the gas. Since the suspension particles are extremely fine, it is often that the suspension particles are inhaled into the lung by passing through the nose hair inside the nasal cavity of human's body. As a result, inflammation of the lungs, asthma or cardiovascular diseases are caused. Furthermore, if the suspension particles are attached with other pollutants, it will be more harmful to the respiratory system of human's body. Recently, the problem of the gas pollution is getting worse, especially, the concentration data of fine suspended particles, e.g., PM2.5, is often too high. Therefore, the detection of the concentration of the suspension particles is getting more attention. However, since the gas flows unstably owing to the wind direction and air volume, and the conventional air quality monitoring stations used for detecting the suspension particles are fixedly disposed at certain locations, people cannot check the concentration of the suspension particles in the surrounding environment. Patent document US-11635221-B describes an exemplary air purifier comprising an airflow generator to generate an airflow through a filter in a first operation mode and an airflow that does not pass through the filter in a second operation mode, and a controller configured receiving sensor information including at least one of an image, a sound, or an air pollution degree of an indoor environment from at least one sensor.

Moreover, people pay more attention to the quality of the air around their lives. For example, carbon monoxide, carbon dioxide, volatile organic compounds (VOC), PM2.5, nitric oxide, sulfur monoxide and even the suspended particles contained in the air are exposed in the environment to affect the human health, and even endanger the life seriously. Therefore, the quality of environmental air has attracted the attention of various countries. How to detect the air quality and avoid the harm from the area with poor air quality is a problem that urgently needs to be solved.

In order to confirm the quality of the air, it is feasible to use a gas detector to detect the air surrounding in the environment. If the detection information is provided in real time to warn the people in the environment, it is helpful of avoiding the harm and facilitates the people to escape the hazard immediately. Thus, it prevents the hazardous gas exposed in the environment from affecting the human health and causing the harm. Therefore, it is a very good application to use a gas detector to detect the air in the surrounding environment.

However, it is not easy to control the indoor air quality. In addition to the air quality of the outdoor space, the air environmental conditions and pollution sources are the major factors that affect indoor air quality. There is needs of intelligently and quickly detecting the indoor air pollution sources in various indoor fields, effectively removing the indoor air pollution to form a clean and safe breathing gas state, instantly monitoring the indoor air quality anytime and anywhere, and quickly purifying the indoor air when the indoor air quality is poor. The main subjects of research and development of the disclosure are to intelligently generate an air convection in the indoor space, quickly detect and determine the location of air pollution field, use the location to effectively control multiple filtering devices to implement the intelligent air convection to accelerate the directional flow of the air pollution, filter and remove the indoor air pollution sources, and make the indoor air pollution treatment of positioning the air pollution positioning- guiding the air pollution guiding-purifying the air pollution completely, whereby a clean and safe breathing gas state is achieved.

### SUMMARY OF THE INVENTION

One object of the present invention is to provide an indoor air pollution prevention system according to claim 1. Since the indoor air pollution occurs and moves at any time, the indoor air pollution prevention system of the present disclosure includes a plurality of gas detectors, at least one filter screen and at least one air guiding device arranged in various indoor fields. With the arrangement, the gas detector determines a characteristic, a concentration and a location of an air pollution, and outputs to form air pollution data, and then the cloud computing service device receives the air pollution data, stores the air pollution data in an air pollution database, implements an artificial intelligence calculation to determine the location of the air pollution, and issues a control command to the air guiding device to control an activation operation of the air guiding device, so that a directional airflow is generated to quickly guide the air pollution to the filter screen for filtering and removal completely. In that, the indoor air pollution treatment of positioning the air pollution-guiding the air pollution-purifying the air pollution completely is formed, and a clean and safe breathing gas state is achieved.

In accordance with an aspect of the present invention, an indoor air pollution prevention system is provided and includes at least one indoor field unit, at least one outdoor field unit, a plurality of gas detectors, at least one filter screen, at least one air guiding device and a cloud computing service device. The indoor field unit is a space surrounded and isolated by a plurality of partitions. The plurality of gas detectors, the at least one filter screen and the at least one air guiding device are disposed inside the indoor field unit. Each of the plurality of gas detectors detects a characteristic, a concentration and a location of an air pollution, and outputs to form air pollution data. The filter screen filters the air pollution in air passing therethrough. The air guiding device guides the air pollution to pass through the filter screen for filtering and removal. The at least one outdoor field unit includes at least one gas detector disposed therein. The cloud computing service device, receives the air pollution data detected in the indoor field unit and the outdoor field unit, stores the air pollution data in an air pollution database, implements an artificial intelligence calculation to determine the location of the air pollution, issues a control command to the air guiding device to control an activation operation of the air guiding device closest to the location of the air pollution, and then controls activation operations of other air guiding devices. Whereby, a directional airflow is generated, and the air containing the air pollution is guided quickly to the filter screen for filtering and removal to reach a gas state of complete purification.

### BRIEF DESCRIPTION OF THE DRAWINGS

The above contents of the present invention will become more readily apparent to those ordinarily skilled in the art after reviewing the following detailed description and accompanying drawings, in which:
FIG. 1A is a schematic view illustrating an indoor air pollution prevention system used in an indoor field unit according to a preferred embodiment of the present invention;
FIG. 1B is a schematic view illustrating an indoor air pollution prevention system used in a plurality of indoor field units according to a preferred embodiment of the present invention;
FIG. 1C is an air pollution removal curve illustrating the indoor air pollution prevention system used in the indoor field unit according to the preferred embodiment of the present invention;
FIG. 2A is a schematic view illustrating the combination of the air guiding devices and the filter screen of the indoor air pollution prevention system according to the embodiment of the present invention;
FIG. 2B is a schematic view illustrating the filter screen of the indoor air pollution prevention system according to the embodiment of the present invention;
FIG. 3A is a schematic perspective view illustrating the gas detector according to the embodiment of the present invention;
FIG. 3B is a schematic perspective view illustrating the gas detector according to the embodiment of the present invention and taken from another perspective;
FIG. 3C is a schematic perspective view illustrating the gas detection module installed inside the gas detector according to the embodiment of the present invention;
FIG. 4A is a schematic perspective view (1) illustrating the gas detection main part according to the embodiment of the present invention;
FIG. 4B is a schematic perspective view (2) illustrating the gas detection main part according to the embodiment of the present invention;
FIG. 4C is an exploded view illustrating the gas detector according to the embodiment of the present invention;
FIG. 5A is a schematic perspective view (1) illustrating the base according to the embodiment of the present invention;
FIG. 5B is a schematic perspective view (2) illustrating the base according to the embodiment of the present invention;
FIG. 6 is a schematic view (3) illustrating the base according to the embodiment of the present invention;
FIG. 7A is a schematic exploded view illustrating the combination of the piezoelectric actuator and the base according to the embodiment of the present invention;
FIG. 7B is a schematic perspective view illustrating the combination of the piezoelectric actuator and the base according to the embodiment of the present invention;
FIG. 8A is a schematic exploded view (1) illustrating the piezoelectric actuator according to the embodiment of the present invention;
FIG. 8B is a schematic exploded view (2) illustrating the piezoelectric actuator according to the embodiment of the present invention;
FIG. 9A is a schematic cross-sectional view (1) illustrating an action of the piezoelectric actuator according to the embodiment of the present invention;
FIG. 9B is a schematic cross-sectional view (2) illustrating an action of the piezoelectric actuator according to the embodiment of the present invention;
FIG. 9C is a schematic cross-sectional view (3) illustrating an action of the piezoelectric actuator according to the embodiment of the present invention;
FIG. 10A is a schematic cross-sectional view (1) illustrating the gas detection main part according to the embodiment of the present invention;
FIG. 10B is a schematic cross-sectional view (2) illustrating the gas detection main part according to the embodiment of the present invention;
FIG. 10C is a schematic cross-sectional view (3) illustrating the gas detection main part according to the embodiment of the present invention;
FIG. 11 is a schematic diagram illustrating the communication transmission of the gas detector according to the embodiment of the present invention; and
FIG. 12 is a schematic diagram of the architecture of the cloud computing service device according to the embodiment of the present invention.

### DETAILED DESCRIPTION OF THE PREFERRED EMBODIMENT

The present invention, also referred with the term disclosure in the following passages, will now be described more specifically with reference to the following embodiments. It is to be noted that the following descriptions of preferred embodiments of this invention are presented herein for purpose of illustration and description only. It is not intended to be exhaustive or to be limited to the precise form disclosed.

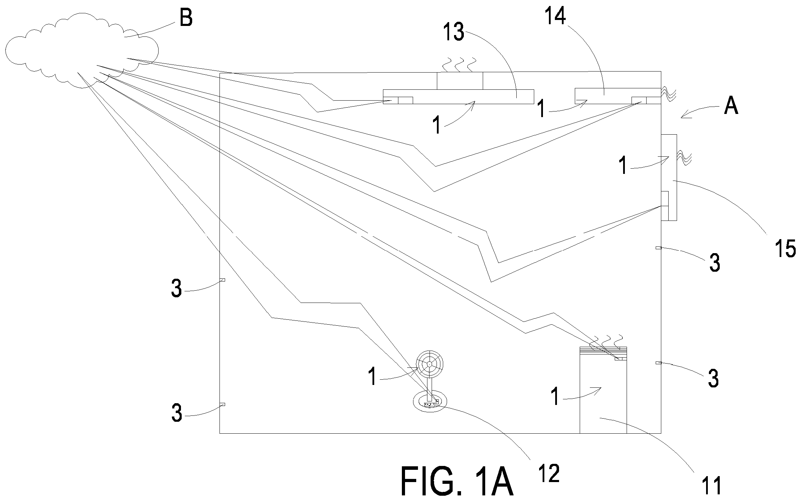

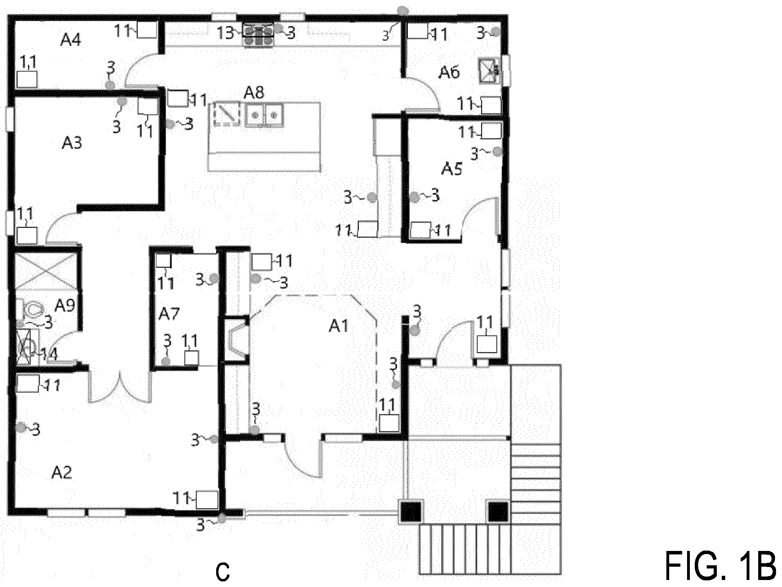

Please refer to FIG. 1A and FIG. 1B. The present disclosure provides an indoor air pollution prevention system includes at least one indoor field unit A, an outdoor field unit C and a cloud computing service device B. Notably, in the embodiment, the indoor field unit A is a space surrounded and isolated by a plurality of partitions. Preferably but not exclusively, in the embodiment, the indoor field unit A is an indoor space formed in a general residential building, and includes a living room A1, a bedroom A2, family room A3, an office A4, a conference room A5, a tea room A6, dressing room A7, a kitchen A8, and a bathroom A9 (as shown in FIG. 1B). Preferably but not exclusively, the indoor field unit A of the indoor air pollution prevention system of the present invention includes all spaces separated in the indoor. In some embodiments, the indoor field unit A is an indoor space formed in a public building, including gymnasium, a concert hall, a theater, an exhibition space, a hospital spaces, an airport space and a station spaces, but limited thereto.

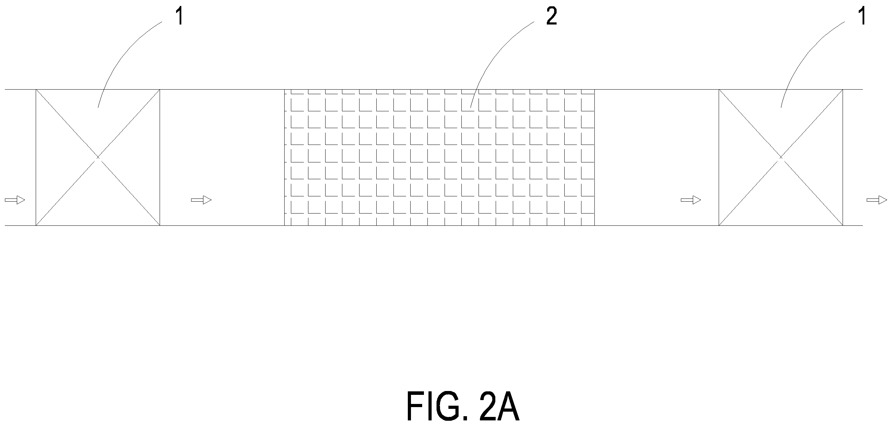

Please refer to FIG. 1A, FIG. 1B and FIG. 2A. In the embodiment, at least one air guiding device 1, at least one filter screen 2 and a plurality of gas detectors 3. The air guiding device 1 has the functions of pumping or supplying air to transport gas in two directions. In the embodiment, the direction of the airflow path for pumping and supplying is indicated by the arrow for illustration (such as the direction indicated by the arrow shown in FIG. 2A). The air guiding device 1 is disposed at the front side of the filter screen 2, or the air guiding device 1 is disposed at the rear side of the filter screen 2. As shown in FIG. 2A, the air guiding devices 1 are arranged at the front and rear sides of the filtering screen 2. Notably, the air guiding device 1 can be an air purifier (including a circulating fan purifier), a fan 12, a range hood 13, an exhaust fan 14 or a fresh air fan 15.

Please refer to FIG. 1B. In the embodiment, the outdoor field unit C includes at least one gas detector 3 disposed therein. The gas detector 3 in the outdoor field unit C is used for detecting a characteristic, a concentration and a location of an air pollution, and outputting to form air pollution data.

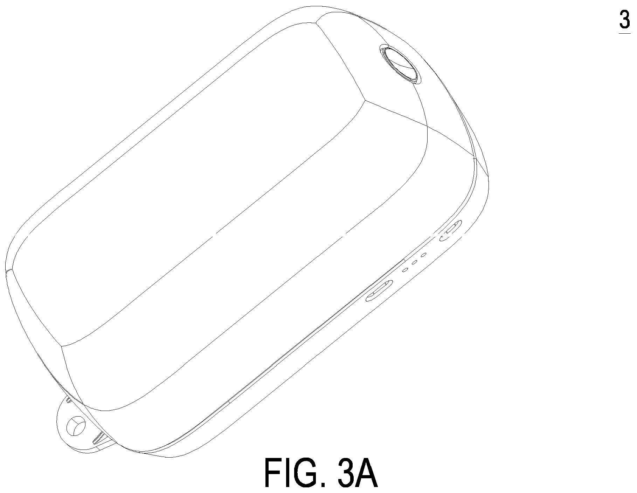

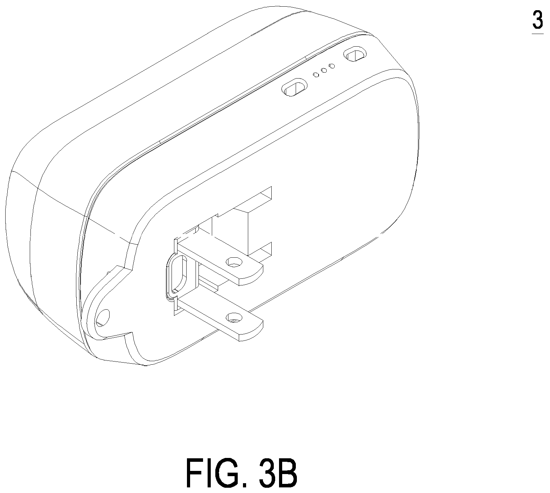

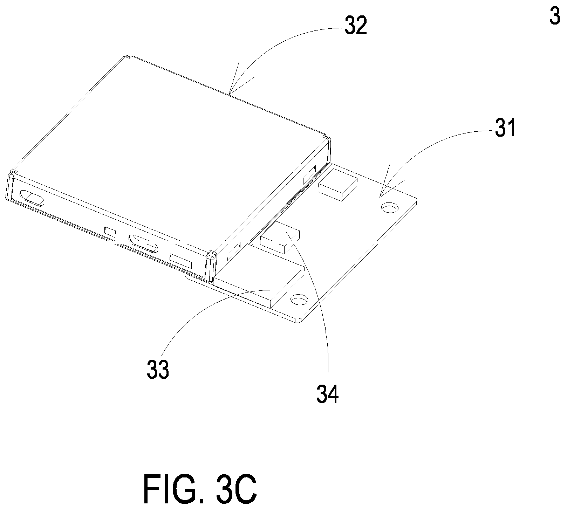

Please refer to FIG. 1A, FIG. 1B, FIG. 3A and FIG. 3B. Each of the plurality of gas detectors 3 disposed in the indoor field unit A and the outdoor field unit C detects a characteristic, a concentration and a location of an air pollution, and outputs to form air pollution data detected in the indoor field unit A and the outdoor field unit C. The filter screen 2 filters the air pollution in air passing therethrough. The air guiding device 1 guides the air pollution to pass through the filter screen 2 for filtering and removal. The cloud computing service device B receives the air pollution data detected in the indoor field unit A and the outdoor field unit C, stores the air pollution data in an air pollution database, implements an artificial intelligence calculation to determine the location of the air pollution, and issuing a control command to the air guiding device to control an activation operation of the air guiding device 1. Whereby, a directional airflow is generated, and the air containing the air pollution is guided quickly to the filter screen 2 for filtering and removal to reach a gas state of complete purification.

Notably, in the above embodiment, the air pollution is at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof.

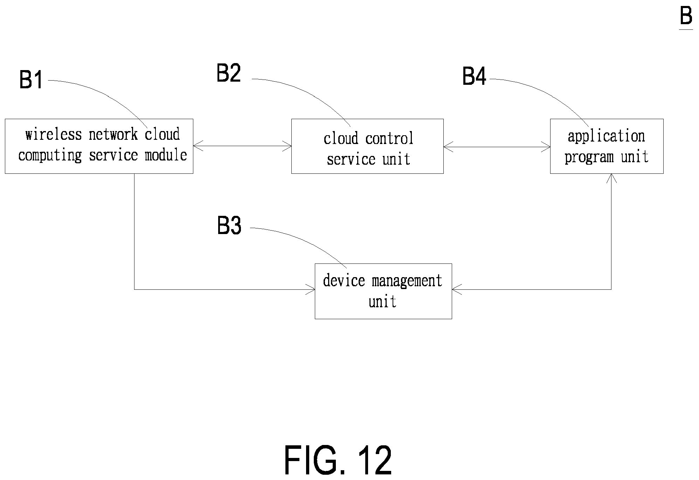

Please refer to FIG. 12. In the embodiment, the cloud computing server device B includes a wireless network cloud computing service module B1, a cloud control service unit B2, a device management unit B3 and an application program unit B4. The wireless network cloud computing service module B1 receives the air pollution data detected in the indoor field unit A, receives the communication information of the air guiding device 1 and transmits the control commands. Moreover, the wireless network cloud computing service module B1 receives the air pollution data detected in the indoor field unit A and transmits the air pollution data to the cloud control service unit B2 to store and form an air pollution database. An artificial intelligence calculation is implemented to determine the location of the air pollution through the air pollution database comparison, so that the control commend is transmitted to the wireless network cloud computing service module B1, and then transmitted to the air guiding device 1 to control the actuation operation through the wireless network cloud computing service module B1. The device management unit B3 receives the communication information of the air guiding device 1 through the wireless network cloud computing service module B1 to manage the user login and device binding. The device management information can be provided to the application program unit B4 for system control and management, and the application program unit B4 can also display and inform the air pollution data obtained by the cloud control service unit B2. The user can know the real-time status of air pollution removal through the mobile phone or the communication device. Moreover, the user can control the operation of the indoor air pollution prevention system through the application program unit B4 of the mobile phone or the communication device.

From the above, the plurality of gas detectors 3 are disposed in the indoor field unit A to detect the characteristics and the concentrations of the air pollution. Preferably but not exclusively, the indoor field unit A is one selected from the group consisting of a living room A1, a bedroom A2, a family room A3, an office A4, a conference room A5, a tea room A6, a dressing room A7, a gymnasium, a concert hall, a theater, an exhibition space, a hospital space, an airport space, a station space and a combination thereof. The cloud computing service device B receives and compares the air pollution data detected by the plurality of gas detectors 3 in the indoor field unit A and the outdoor field unit C. If the air pollution data detected in the indoor field unit A is higher than the air pollution data detected in the outdoor field unit C, the cloud computing service device B issues the control command to the air guiding device 1 for the activation operation, the air in the outdoor field unit C is introduced through the filter screen 2 for filtering and enters into the indoor field unit A, and the air pollution in the indoor field unit is A guided to the filter screen 2 for filtering and removal to the outdoor field unit C, thereby the air in the indoor field unit A is exchanged to reach the gas state of complete purification. Notably, the air guiding device 1 is a fresh air fan 15 (gas exchanging device), and the filter screen 2 is directly disposed within the air guiding device 1 to filter the air pollution.

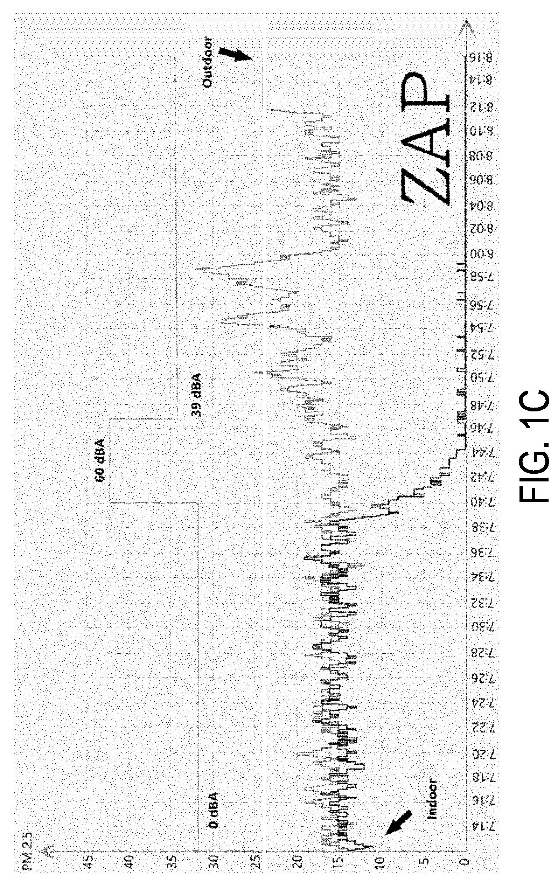

Preferably but not exclusively, the indoor field unit A is one selected from the group consisting of a living room A1, a bedroom A2, a family room A3, an office A4, a conference room A5, a tea room A6, a dressing room A7, a gymnasium, a concert hall, a theater, an exhibition space, a hospital space, an airport space, a station space and a combination thereof. The cloud computing service device B receives and compares at least two or more of the air pollution data detected by the plurality of gas detectors 3 in the indoor field unit A, intelligently calculates to position the location of the air pollution in the indoor field unit A, and intelligently selects to issue the control command to the air guiding device 1. In that, the air guiding device 1 closest to the location of the air pollution is enabled for the activation operation firstly, then other air guiding devices 1 are enabled for the activation operation, and the directional airflow is generated to guide the air pollution to the filter screen 2 for filtering and removal. Thereby, the air pollution in the indoor field unit A is cleaned quickly to reach the gas state of complete purification. As shown in FIG. 1C, the gas detector 3 installed in the indoor field unit A for detecting suspended particles PM2.5 is taken as an example. Before the user activates the indoor air pollution prevention system at 7:40, the PM2.5 value of suspended particulate matter detected in the indoor field unit A is similar to the PM2.5 value of suspended particulate matter detected in the outdoor field unit C. When the indoor air pollution prevention system is activated at 7:40, the gas detector 3 in the indoor field unit A detects the air pollution data of suspended particulate matter PM2.5, and the cloud computing service device B receives and compares at least two or more of the air pollution data detected by the plurality of gas detectors 3 in the indoor field unit A, intelligently calculates to position the location of the air pollution in the indoor field unit A, and intelligently selects to issue the control command to the air guiding device 1. At 7:44, it can be seen that the value of the air pollution data detected in the entire indoor field unit A is dropped rapidly, and the effect of air pollution complete purification is maintained thereafter. Notably, in the embodiment, the air guiding device 1 in the indoor field unit A can be an air purifier 11 (including a circulating fan purifier) or a fan 12, and the filter screen 2 can also be directly disposed within the air guiding device 1 to filter the air pollution.

In an embodiment, the indoor field unit A is a kitchen field unit A8, and the cloud computing service device B receives and compares the air pollution data detected by the gas detector 3 in the indoor field unit A. When the air pollution data exceeds a safety detection value, the control command is intelligently selected and issued to the air guiding device 1 for the activation operation, and the air pollution is quickly guided to the filter screen 2 for filtering and removal, so that the air pollution in the indoor field unit A is cleaned to reach the gas state of complete purification. Notably, in the embodiment, the air guiding device 1 in the indoor field unit A is a range hood 13. The filter screen 2 is directly disposed within the air guiding device 1 to the filter air pollution. Moreover, the gas detector 3 is also directly disposed within the air guiding device 1.

In an embodiment, the indoor field unit A is a bathroom field unit A9, and the cloud computing service device B receives and compares the air pollution data detected by the gas detector 3 in the indoor field unit A. When the air pollution data exceeds a safety detection value, the control command is intelligently selected and issued to the air guiding device 1 for the activation operation, and the air pollution is quickly guided to the filter screen 2 for filtering and removal, so that the air pollution in the indoor field unit A is cleaned to reach the gas state of complete purification, and the temperature and the humidity of the indoor field unit A are controlled. Notably, the air guiding device 1 in the indoor field unit A is an exhaust fan 14, the filter screen 2 is directly disposed within the air guiding device 1 to filter the air pollution, and the gas detector 3 is also directly disposed on the air guiding device 1.

Notably, in the above embodiments, the safety detection value includes at least one selected from the group consisting of a concentration of PM2.5 which is less than 15 µg/m³, a concentration of carbon dioxide which is less than 1000 ppm, a concentration of total volatile organic compounds (TVOC) which is less than 0.56 ppm, a concentration of formaldehyde (HCHO) which is less than 0.08 ppm, a colony-forming unit of bacteria which is less than 1500 CFU/m³, a colony-forming unit of fungi which is less than 1000 CFU/m³, a concentration of sulfur dioxide which is less than 0.075 ppm, a concentration of nitrogen dioxide which is less than 0.1 ppm, a concentration of carbon monoxide which is less than 9 ppm, a concentration of ozone which is less than 0.06 ppm, and a concentration of lead which is less than 0.15 µg/m³.

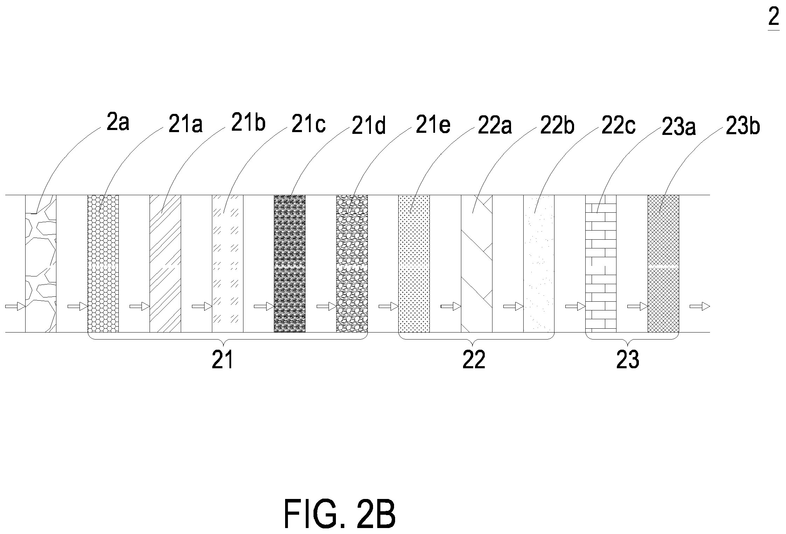

Please refer to FIG. 2B, again. In the embodiment, the filter screen 2 is a filter screen to clean the air pollution through a physical way of blocking and absorbing. Preferably but not exclusively, the filter screen is a high efficiency particulate air (HEPA) filter screen 2a, which is configured to absorb the chemical smoke, the bacteria, the dust particles and the pollen contained in the air pollution, so that the air pollution introduced is filtered and purified to achieve the effect of filtering and purification. Preferably but not exclusively, the filter screen 2 is a high HEPA filter screen 2a coated with decomposition layer 21 to clean the air pollution through a chemical way. Preferably but not exclusively, the decomposition layer 21 includes an activated carbon 21a configured to remove organic and inorganic substances in air pollution, and remove colored and odorous substances. Preferably but not exclusively, the decomposition layer 21 includes a cleansing factor containing chlorine dioxide layer 21b configured to inhibit viruses, bacteria, fungi, influenza A, influenza B, enterovirus and norovirus in the air pollution, and the inhibition ratio can reach 99% and more, thereby reducing the cross-infection of viruses. Preferably but not exclusively, the decomposition layer 21 includes an herbal protective layer 21c extracted from ginkgo and Japanese Rhus chinensis configured to resist allergy effectively and destroy a surface protein of influenza virus (such as H1N1 influenza virus) passing therethrough. Preferably but not exclusively, the decomposition layer 21 includes a silver ion 21d configured to inhibit viruses, bacteria and fungi contained in the air pollution. Preferably but not exclusively, the decomposition layer 21 includes a zeolite 21e configured to remove ammonia nitrogen, heavy metals, organic pollutants, Escherichia coli, phenol, chloroform and anionic surfactants. In some embodiments, the filter screen 2 is a high HEPA filter screen 2a combined with a light irradiation element 22 to clean the air pollution through a chemical way. Preferably but not exclusively, the light irradiation element 22 is a photo-catalyst unit including a photo catalyst 22a and an ultraviolet lamp 22b. When the photo catalyst 22a is irradiated by the ultraviolet lamp 22b, the light energy is converted into the chemical energy, thereby decomposes harmful gases and disinfects bacteria contained in the air pollution, so as to achieve the effects of filtering and purifying. Preferably but not exclusively, the light irradiation element 22 is a photo-plasma unit including a nanometer irradiation tube 22c. When the introduced air pollution is irradiated by the nanometer irradiation tube 22c, the oxygen molecules and water molecules contained in the air pollution are decomposed into high oxidizing photo-plasma, and an ion flow capable of destroying organic molecules is generated. In that, volatile formaldehyde, volatile toluene and volatile organic compounds (VOC) contained in the air pollution are decomposed into water and carbon dioxide, so as to achieve the effects of filtering and purifying. In some embodiments, the filter screen 2 is a high HEPA filter screen 2a combined with a decomposition unit 23 to clean the air pollution through a chemical way. Preferably but not exclusively, the decomposition unit is a negative ion unit 23a with s a dust collecting plate. It makes the suspended particles in the air pollution to carry with positive charge and adhered to the dust collecting plate carry with negative charges, so as to achieve the effects of filtering and purifying. Preferably but not exclusively, the decomposition unit is a plasma ion unit 23b. The oxygen molecules and water molecules contained in the air pollution are decomposed into positive hydrogen ions (H⁺) and negative oxygen ions (O²⁻) by the plasma ion. The substances attached with water around the ions are adhered on the surface of viruses and bacteria and converted into OH radicals with extremely strong oxidizing power, thereby removing hydrogen (H) from the protein on the surface of viruses and bacteria, and thus decomposing (oxidizing) the protein, so as to filter the introduced air pollution and achieve the effects of filtering and purifying.

For understanding the implementation of the indoor air pollution prevention system of the present disclosure, the internal structure and function of the gas detector 3 will be described below.

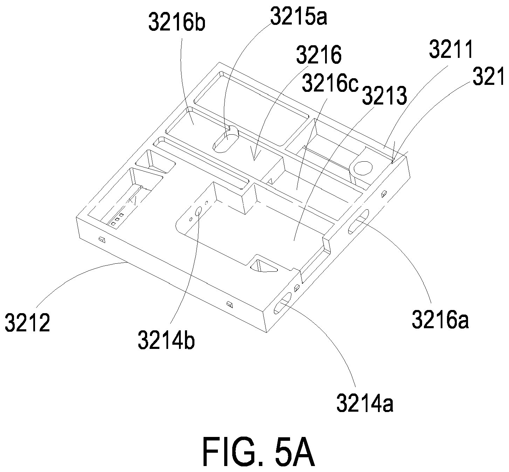

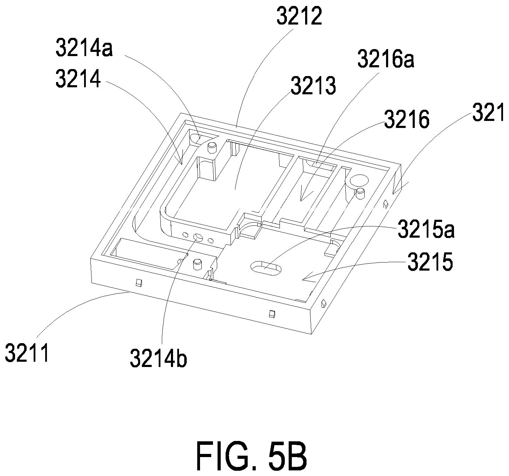

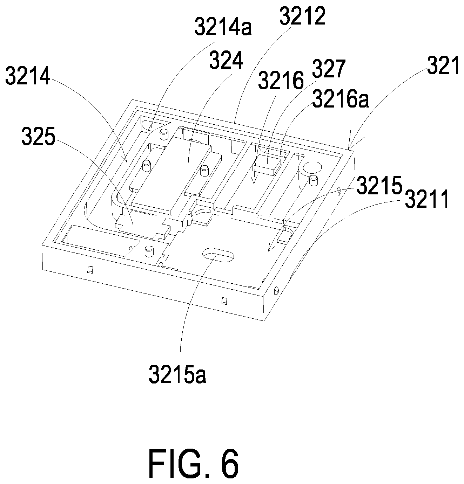

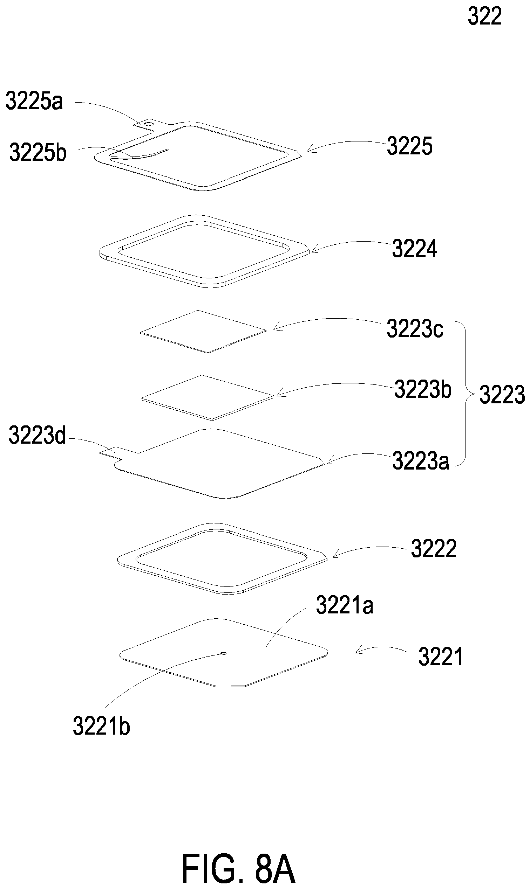

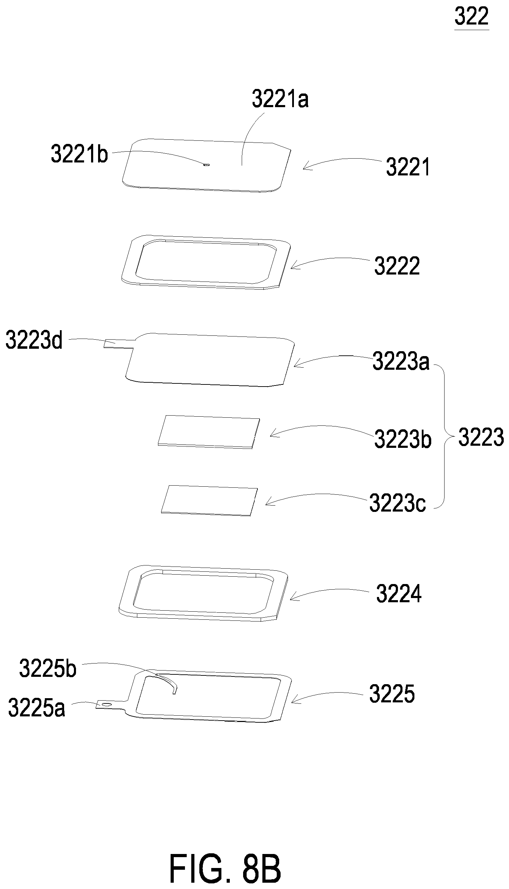

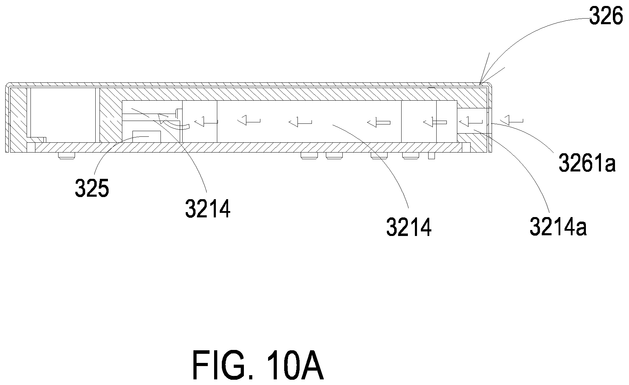

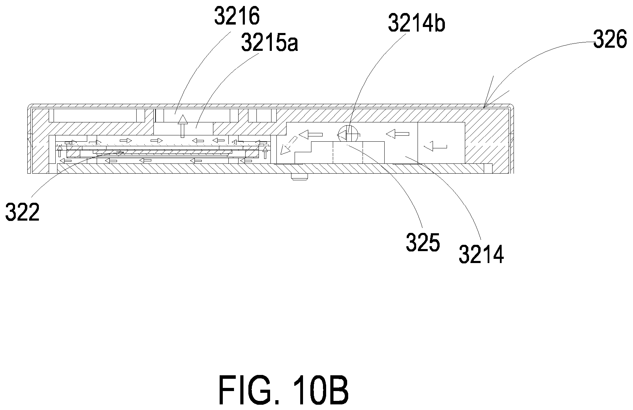

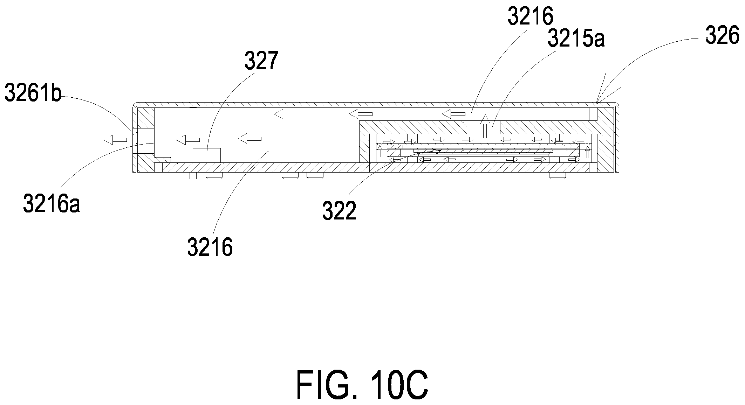

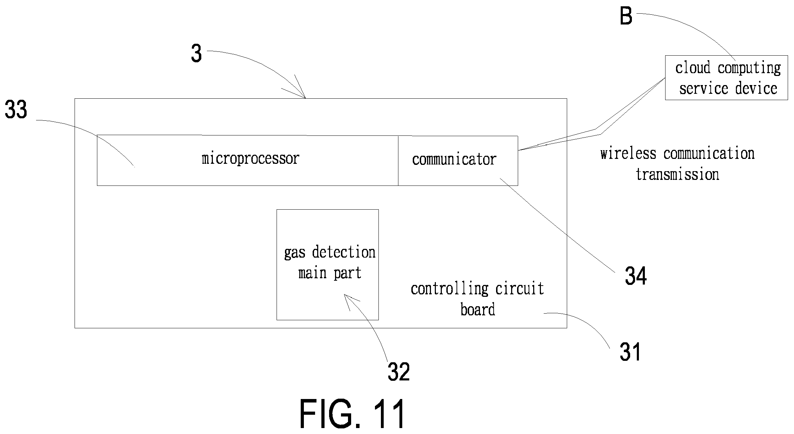

Please refer to FIG. 3A to FIG. 11. In the present disclosure, the gas detector 3 is described with the symbol 3 below. The gas detector 3 includes a gas detection module disposed thereon. The gas detection module includes a controlling circuit board 31, a gas detection main part 32, a microprocessor 33 and a communicator 34. In the embodiment, the gas detection main part 32, the microprocessor 33 and the communicator 34 are integrally packaged on the controlling circuit board 31 and electrically connected to the controlling circuit board 31. The microprocessor 33 and the communicator 34 are disposed on the controlling circuit board 31, and the microprocessor 33 controls the detection of the gas detection main part 32. In that, the gas detection main part 32 detects the air pollution and outputs a detection signal, and the microprocessor 33 receives and processes the detection signal to generate air pollution data and provides the air pollution data to the communicator 34 for a wireless communication transmission externally to the cloud computing service device B.

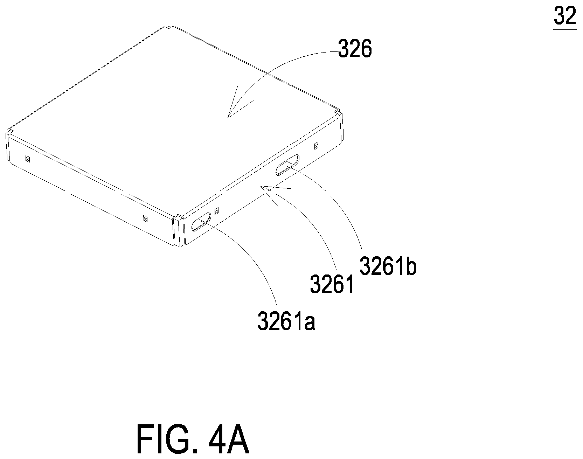

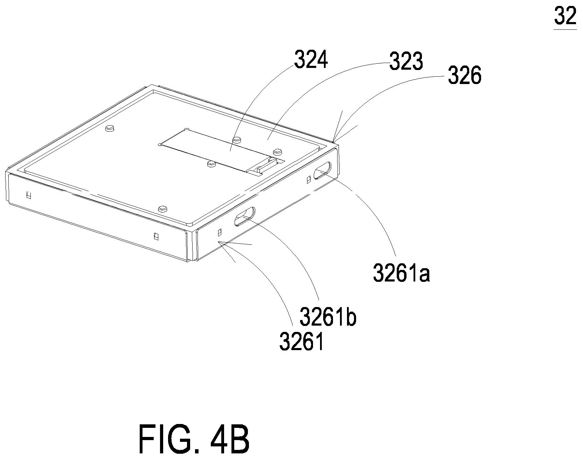

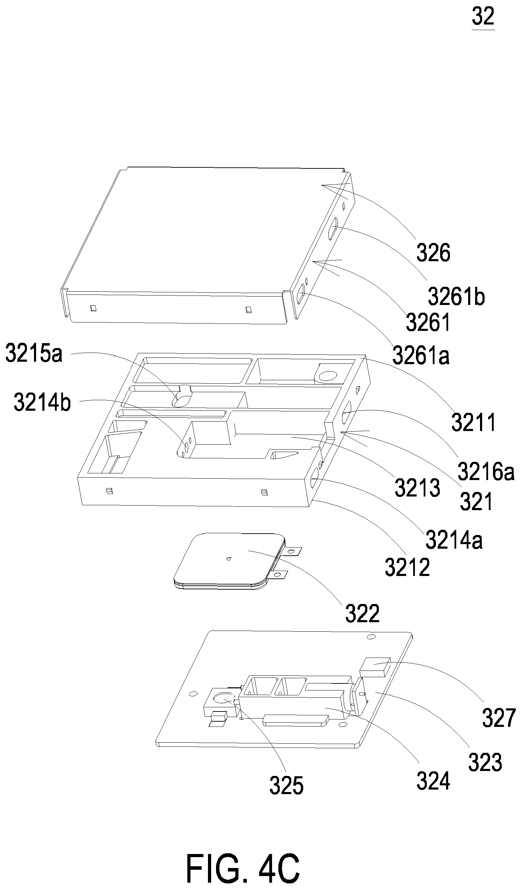

Please refer to FIG. 4A to FIG. 9A. In the embodiment, the gas detection main part 32 includes a base 321, a piezoelectric actuator 322, a driving circuit board 323, a laser component 324, a particulate sensor 325, and an outer cover 326. In the embodiment, the base 321 includes a first surface 3211, a second surface 3212, a laser loading region 3213, a gas-inlet groove 3214, a gas-guiding-component loading region 3215 and a gas-outlet groove 3216. The first surface 3211 and the second surface 3212 are two surfaces opposite to each other. In the embodiment, the laser loading region 3213 is hollowed out from the first surface 3211 toward the second surface 3212. The outer cover 326 covers the base 321 and includes a side plate 3261. The side plate 3261 has an inlet opening 3261a and an outlet opening 3261b. The gas-inlet groove 3214 is concavely formed from the second surface 3212 and disposed adjacent to the laser loading region 3213. The gas-inlet groove 3214 includes a gas-inlet 3214a and two lateral walls. The gas-inlet 3214a is in communication with an environment outside the base 321, and is spatially corresponding in position to an inlet opening 3261a of the outer cover 326. Two transparent windows 3214b are opened on the two lateral walls of the gas-inlet groove 3214 and are in communication with the laser loading region 3213. Therefore, the first surface 3211 of the base 321 is covered and attached by the outer cover 326, and the second surface 3212 is covered and attached by the driving circuit board 323, so that an inlet path is defined by the gas-inlet groove 3214.

In the embodiment, the gas-guiding-component loading region 3215 mentioned above is concavely formed from the second surface 3212 and in communication with the gas-inlet groove 3214. A ventilation hole 3215a penetrates a bottom surface of the gas-guiding-component loading region 3215. The gas-guiding-component loading region 3215 includes four positioning protrusions 3215b disposed at four corners of the gas-guiding-component loading region 3215, respectively. In the embodiment, the gas-outlet groove 3216 includes a gas-outlet 3216a, and the gas-outlet 3216a is spatially corresponding to the outlet opening 3261b of the outer cover 326. The gas-outlet groove 3216 includes a first section 3216b and a second section 3216c. The first section 3216b is concavely formed out from the first surface 3211 in a region spatially corresponding to a vertical projection area of the gas-guiding-component loading region 3215. The second section 3216c is hollowed out from the first surface 3211 to the second surface 3212 in a region where the first surface 3211 is extended from the vertical projection area of the gas-guiding-component loading region 3215. The first section 3216b and the second section 3216c are connected to form a stepped structure. Moreover, the first section 3216b of the gas-outlet groove 3216 is in communication with the ventilation hole 3215a of the gas-guiding-component loading region 3215, and the second section 3216c of the gas-outlet groove 3216 is in communication with the gas-outlet 3216a. In that, when first surface 3211 of the base 321 is attached and covered by the outer cover 326 and the second surface 3212 of the base 321 is attached and covered by the driving circuit board 323, the gas-outlet groove 3216 and the driving circuit board 323 collaboratively define an outlet path.

In the embodiment, the laser component 324 and the particulate sensor 325 are disposed on and electrically connected to the driving circuit board 323 and located within the base 321. In order to clearly describe and illustrate the positions of the laser component 324 and the particulate sensor 325 in the base 321, the driving circuit board 323 is intentionally omitted. The laser component 324 is accommodated in the laser loading region 3213 of the base 321, and the particulate sensor 325 is accommodated in the gas-inlet groove 3214 of the base 321 and is aligned to the laser component 324. In addition, the laser component 324 is spatially corresponding to the transparent window 3214b, therefore, a light beam emitted by the laser component 324 passes through the transparent window 3214b and is irradiated into the gas-inlet groove 3214. A light beam path emitted from the laser component 324 passes through the transparent window 3214b and extends in an orthogonal direction perpendicular to the gas-inlet groove 3214. In the embodiment, a projecting light beam emitted from the laser component 324 passes through the transparent window 3214b and enters the gas-inlet groove 3214 to irradiate the suspended particles contained in the gas passing through the gas-inlet groove 3214. When the suspended particles contained in the gas are irradiated and generate scattered light spots, the scattered light spots are received and calculated by the particulate sensor 325 to obtain the gas detection data. In addition, the gas sensor 327 is positioned and disposed on the driving circuit board 323, electrically connected to the driving circuit board 323, and accommodated in the gas-outlet groove 3216, so as to detect the air pollution introduced into the gas-outlet groove 3216. In other embodiments, the gas sensor 327 is a volatile-organic-compound sensor, a formaldehyde sensor, a bacteria sensor, a virus sensor or a combination thereof, the volatile-organic-compound sensor is used for detecting gas information of carbon dioxide (CO₂) or volatile organic compounds (TVOC), the formaldehyde sensor is used for detecting gas information of formaldehyde (HCHO), the bacteria sensor is used for detecting gas information of bacteria or fungi, and the virus sensor used for detecting gas information of virus.

In the embodiment, the piezoelectric actuator 322 is accommodated in the square-shaped gas-guiding-component loading region 3215 of the base 321. In addition, the gas-guiding-component loading region 3215 of the base 321 is in fluid communication with the gas-inlet groove 3214. When the piezoelectric actuator 322 is enabled, the gas in the gas-inlet groove 3214 is inhaled by the piezoelectric actuator 322, so that the gas flows into the piezoelectric actuator 322, and is transported into the gas-outlet groove 3216 through the ventilation hole 3215a of the gas-guiding-component loading region 3215. Moreover, the driving circuit board 323 covers the second surface 3212 of the base 321, and the laser component 324 is disposed on the driving circuit board 323, and is electrically connected to the driving circuit board 323. The particulate sensor 325 is also disposed on the driving circuit board 323 and electrically connected to the driving circuit board 323. In that, when the outer cover 326 covers the base 321, the inlet opening 3261a is spatially corresponding to the gas-inlet 3214a of the base 321, and the outlet opening 3261b is spatially corresponding to the gas-outlet 3216a of the base 321.

In the embodiment, the piezoelectric actuator 322 includes a gas-injection plate 3221, a chamber frame 3222, an actuator element 3223, an insulation frame 3224 and a conductive frame 3225. In the embodiment, the gas-injection plate 3221 is made by a flexible material and includes a suspension plate 3221a and a hollow aperture 3221b. The suspension plate 3221a is a sheet structure and is permitted to undergo a bending deformation. Preferably but not exclusively, the shape and the size of the suspension plate 3221a are accommodated in the inner edge of the gas-guiding-component loading region 3215, but not limited thereto. The hollow aperture 3221b passes through a center of the suspension plate 3221a, so as to allow the gas to flow therethrough. Preferably but not exclusively, in the embodiment, the shape of the suspension plate 3221a is selected from the group consisting of a square, a circle, an ellipse, a triangle and a polygon, but not limited thereto.

In the embodiment, the chamber frame 3222 is carried and stacked on the gas-injection plate 3221. In addition, the shape of the chamber frame 3222 is corresponding to the gas-injection plate 3221. The actuator element 3223 is carried and stacked on the chamber frame 3222. A resonance chamber 3226 is collaboratively defined by the actuator element 3223, the chamber frame 3222 and the suspension plate 3221a and is formed between the actuator element 3223, the chamber frame 3222 and the suspension plate 3221a. The insulation frame 3224 is carried and stacked on the actuator element 3223 and the appearance of the insulation frame 3224 is similar to that of the chamber frame 3222. The conductive frame 3225 is carried and stacked on the insulation frame 3224, and the appearance of the conductive frame 3225 is similar to that of the insulation frame 3224. In addition, the conductive frame 3225 includes a conducting pin 3225a and a conducting electrode 3225b. The conducting pin 3225a is extended outwardly from an outer edge of the conductive frame 3225, and the conducting electrode 3225b is extended inwardly from an inner edge of the conductive frame 3225. Moreover, the actuator element 3223 further includes a piezoelectric carrying plate 3223a, an adjusting resonance plate 3223b and a piezoelectric plate 3223c. The piezoelectric carrying plate 3223a is carried and stacked on the chamber frame 3222. The adjusting resonance plate 3223b is carried and stacked on the piezoelectric carrying plate 3223a. The piezoelectric plate 3223c is carried and stacked on the adjusting resonance plate 3223b. The adjusting resonance plate 3223b and the piezoelectric plate 3223c are accommodated in the insulation frame 3224. The conducting electrode 3225b of the conductive frame 3225 is electrically connected to the piezoelectric plate 3223c. In the embodiment, the piezoelectric carrying plate 3223a and the adjusting resonance plate 3223b are made by a conductive material. The piezoelectric carrying plate 3223a includes a piezoelectric pin 3223d. The piezoelectric pin 3223d and the conducting pin 3225a are electrically connected to a driving circuit (not shown) of the driving circuit board 323, so as to receive a driving signal, such as a driving frequency and a driving voltage. Through this structure, a circuit is formed by the piezoelectric pin 3223d, the piezoelectric carrying plate 3223a, the adjusting resonance plate 3223b, the piezoelectric plate 3223c, the conducting electrode 3225b, the conductive frame 3225 and the conducting pin 3225a for transmitting the driving signal. Moreover, the insulation frame 3224 is insulated between the conductive frame 3225 and the actuator element 3223, so as to avoid the occurrence of a short circuit. Thereby, the driving signal is transmitted to the piezoelectric plate 3223c. After receiving the driving signal such as the driving frequency and the driving voltage, the piezoelectric plate 3223c deforms due to the piezoelectric effect, and the piezoelectric carrying plate 3223a and the adjusting resonance plate 3223b are further driven to generate the bending deformation in the reciprocating manner.

Furthermore, in the embodiment, the adjusting resonance plate 3223b is located between the piezoelectric plate 3223c and the piezoelectric carrying plate 3223a and served as a cushion between the piezoelectric plate 3223c and the piezoelectric carrying plate 3223a. Thereby, the vibration frequency of the piezoelectric carrying plate 3223a is adjustable. Basically, the thickness of the adjusting resonance plate 3223b is greater than the thickness of the piezoelectric carrying plate 3223a, and the vibration frequency of the actuator element 3223 can be adjusted by adjusting the thickness of the adjusting resonance plate 3223b.

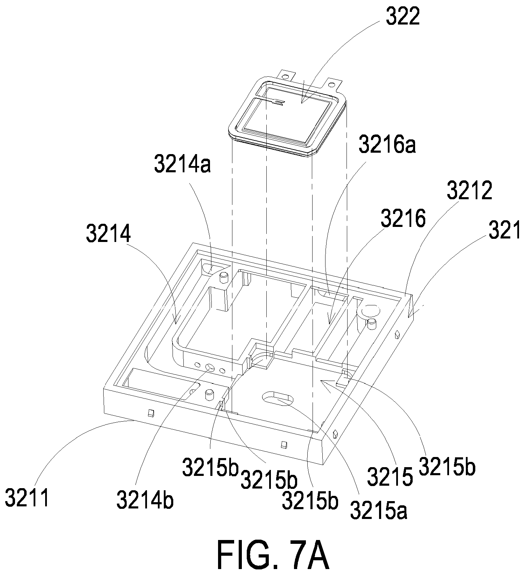

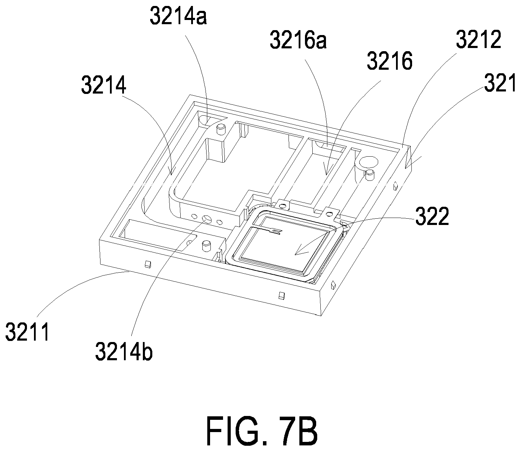

Please further refer to FIG. 7A, FIG. 7B, FIG.8A, FIG. 8B and FIG. 9A. In the embodiment, the gas-injection plate 3221, the chamber frame 3222, the actuator element 3223, the insulation frame 3224 and the conductive frame 3225 are stacked and positioned in the gas-guiding-component loading region 3215 sequentially, so that the piezoelectric actuator 322 is supported and positioned in the gas-guiding-component loading region 3215. A plurality of clearances 3221c are defined between the suspension plate 3221a of the gas-injection plate 3221 and an inner edge of the gas-guiding-component loading region 3215 for gas flowing therethrough. In the embodiment, a flowing chamber 3227 is formed between the gas-injection plate 3221 and the bottom surface of the gas-guiding-component loading region 3215. The flowing chamber 3227 is in communication with the resonance chamber 3226 between the actuator element 3223, the chamber frame 3222 and the suspension plate 3221a through the hollow aperture 3221b of the gas-injection plate 3221. By controlling the vibration frequency of the gas in the resonance chamber 3226 to be close to the vibration frequency of the suspension plate 3221a, the Helmholtz resonance effect is generated between the resonance chamber 3226 and the suspension plate 3221a, so as to improve the efficiency of gas transportation. When the piezoelectric plate 3223c is moved away from the bottom surface of the gas-guiding-component loading region 3215, the suspension plate 3221a of the gas-injection plate 3221 is driven to move away from the bottom surface of the gas-guiding-component loading region 3215 by the piezoelectric plate 3223c. In that, the volume of the flowing chamber 3227 is expanded rapidly, the internal pressure of the flowing chamber 3227 is decreased to form a negative pressure, and the gas outside the piezoelectric actuator 322 is inhaled through the clearances 3221c and enters the resonance chamber 3226 through the hollow aperture 3221b. Consequently, the pressure in the resonance chamber 3226 is increased to generate a pressure gradient. When the suspension plate 3221a of the gas-injection plate 3221 is driven by the piezoelectric plate 3223c to move toward the bottom surface of the gas-guiding-component loading region 3215, the gas in the resonance chamber 3226 is discharged out rapidly through the hollow aperture 3221b, and the gas in the flowing chamber 3227 is compressed, thereby the converged gas is quickly and massively ejected out of the flowing chamber 3227 under the condition close to an ideal gas state of the Benulli's law, and transported to the ventilation hole 3215a of the gas-guiding-component loading region 3215.

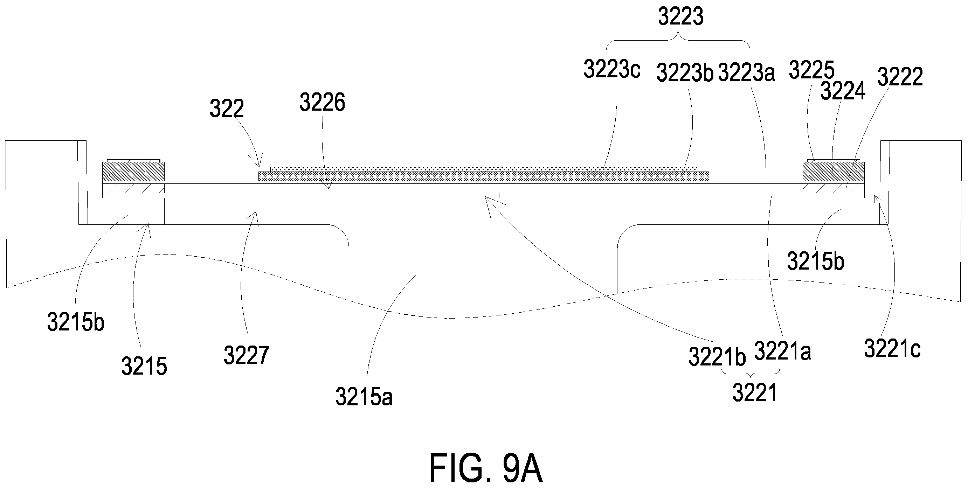

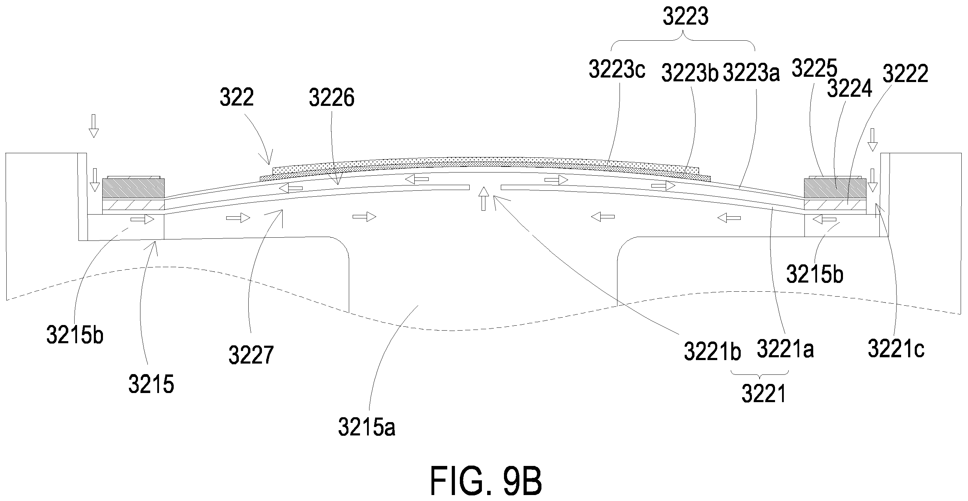

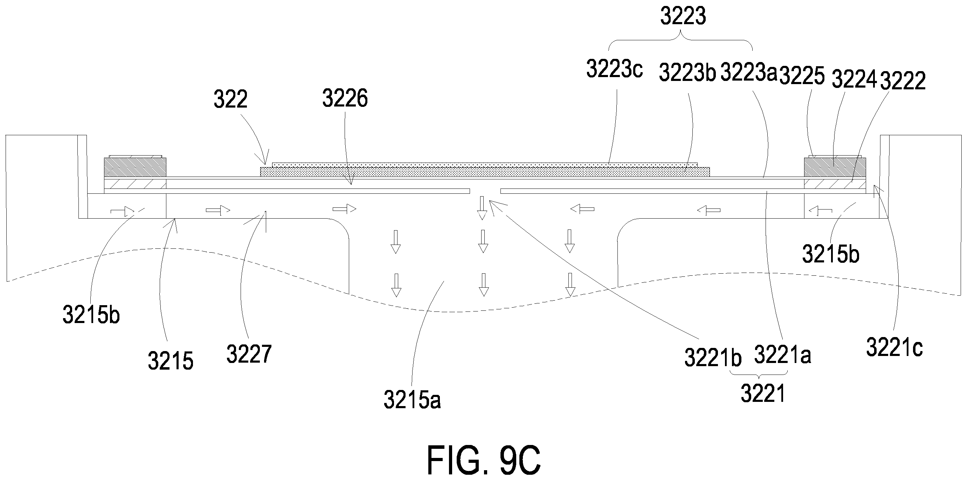

By repeating the above operation steps shown in FIG. 9B and FIG. 9C, the piezoelectric plate 3223c is driven to generate the bending deformation in a reciprocating manner. According to the principle of inertia, since the gas pressure inside the resonance chamber 3226 is lower than the equilibrium gas pressure after the converged gas is ejected out, the gas is introduced into the resonance chamber 3226 again. Moreover, the vibration frequency of the gas in the resonance chamber 3226 is controlled to be close to the vibration frequency of the piezoelectric plate 3223c, so as to generate the Helmholtz resonance effect to achieve the gas transportation at high speed and in large quantities. The gas is inhaled through the gas-inlet 3214a on the outer cover 326, flows into the gas-inlet groove 3214 of the base 321 through the gas-inlet 3214a, and is transported to the position of the particulate sensor 325. The piezoelectric actuator 322 is enabled continuously to inhale the gas into the inlet path, and facilitate the gas outside the gas detection module to be introduced rapidly, flow stably, and transported above the particulate sensor 325. At this time, a projecting light beam emitted from the laser component 324 passes through the transparent window 3214b to irritate the suspended particles contained in the gas flowing above the particulate sensor 325 in the gas-inlet groove 3214. When the suspended particles contained in the gas are irradiated and generate scattered light spots, the scattered light spots are received and calculated by the particulate sensor 325 for obtaining related information about the sizes and the concentration of the suspended particles contained in the gas. Moreover, the gas above the particulate sensor 325 is continuously driven and transported by the piezoelectric actuator 322, flows into the ventilation hole 3215a of the gas-guiding-component loading region 3215, and is transported to the gas-outlet groove 3216. At last, after the gas flows into the gas outlet groove 3216, the gas is continuously transported into the gas-outlet groove 3216 by the piezoelectric actuator 322, and thus the gas in the gas-outlet groove 3216 is pushed to discharge through the gas-outlet 3216a and the outlet opening 3261b.

The gas detector 3 of the present disclosure not only includes particulate sensor 325 for detecting the particulate matters (e.g., PM1 PM2.5 or PM10) in the gas, but also includes a gas sensor for detecting the gas characteristics of the introduced gas, for example, to determine whether the gas is formaldehyde, ammonia, carbon monoxide, carbon dioxide, oxygen, ozone, or the like. Therefore, in one or some embodiments, the gas detector 3 of the present disclosure further includes the gas sensor 327 positioned and disposed on the driving circuit board 323, electrically connected to the driving circuit board 323, and accommodated in the gas-outlet groove 3216, so as to detect the concentration or the characteristics of volatile organic compounds contained in the gas exported from the gas outlet path.

In summary, the present invention provides an indoor air pollution prevention according to claim 1. In order to solve the problem that indoor air pollution occurs at any time and is difficult to control, the indoor air pollution prevention system of the present disclosure includes a plurality of gas detectors, at least one filter screen and at least one air guiding device arranged in various indoor fields. With the arrangement, the gas detector determines a characteristic, a concentration and a location of an air pollution, and outputs to form air pollution data, and then the cloud computing service device receives the air pollution data detected in the indoor field unit and the outdoor field unit, stores the air pollution data in an air pollution database, implements an artificial intelligence calculation to determine the location of the air pollution, and issues a control command to the air guiding device to control an activation operation of the air guiding device, so that a directional airflow is generated to quickly guide the air pollution to the filter screen for filtering and removal completely. In that, the indoor air pollution treatment of positioning the air pollution-guiding the air pollution-purifying the air pollution completely is formed, and a clean and safe breathing gas state is achieved. The present disclosure includes the industrial applicability and the inventive steps.

## Claims

1. An indoor air pollution prevention system comprising:
at least one indoor field unit (A), wherein the indoor field unit (A) is a space surrounded and isolated by a plurality of partitions, and a plurality of gas detectors (3), at least one filter screen (2) and at least one air guiding device (1) are disposed inside the indoor field unit (A), wherein each of the plurality of gas detectors (3) detects a characteristic, a concentration and a location of an air pollution, and outputs to form air pollution data, the filter screen (2) filters the air pollution in air passing therethrough, and the air guiding device (1) guides the air pollution to pass through the filter screen (2) for filtering and removal;
at least one outdoor field unit (C) with at least one gas detector (3) disposed therein; and
**characterized by** further comprising a cloud computing service device (B) configured for:
receiving the air pollution data detected in the indoor field unit (A) and the outdoor field unit (C), storing the air pollution data in an air pollution database, implementing an artificial intelligence calculation to determine the location of the air pollution, issuing a control command to the air guiding device to control an activation operation of the air guiding device (1) closest to the location of the air pollution, and then controlling activation operations of other air guiding devices (a), whereby a directional airflow is generated, and the air containing the air pollution is guided quickly to the filter screen (2) for filtering and removal to reach a gas state of complete purification.

2. The indoor air pollution prevention system according to claim 1, wherein the air pollution is at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof.

3. The indoor air pollution prevention system according to claim 1, wherein the cloud computing service device (B) comprises a wireless network cloud computing service module (B1), a cloud control service unit (B2), a device management unit (B3) and an application program unit (B4).

4. The indoor air pollution prevention system according to claim 1, wherein the indoor field unit (A) is one selected from the group consisting of a living room (A1), a bedroom (A2), a family room (A3), an office (A4), a conference room (A5), a tea room (A6), a dressing room (A7), a gymnasium, a concert hall, a theater, an exhibition space, a hospital space, an airport space, a station space and a combination thereof, and the cloud computing service device (B) receives and compares the air pollution data detected by the plurality of gas detectors (3) in the indoor field unit (A) and the outdoor field unit (C), wherein if the air pollution data detected in the indoor field unit (A) is higher than the air pollution data detected in the outdoor field unit (C), the cloud computing service device (B) issues the control command to the air guiding device (1) for the activation operation, the air in the outdoor field unit (C) is introduced through the filter screen (2) for filtering and enters into the indoor field unit (A), and the air pollution in the indoor field unit (A) is guided to the filter screen (2) for filtering and removal to the outdoor field unit (C), thereby the air in the indoor field unit(A) is exchanged to reach the gas state of complete purification.

5. The indoor air pollution prevention system according to claim 1, wherein the filter screen (2) is disposed within the air guiding device (1) to filter the air pollution, and the air guiding device (1) is one selected from the group consisting of a fresh air fan (11), an air purifier, a circulating fan purifier, a fan (12), a range hood (13), an exhaust fan (14) and a combination thereof.

6. The indoor air pollution prevention system according to claim 1, wherein the indoor field unit (A) is one selected from the group consisting of a living room (A1), a bedroom (A2), a family room (A3), an office (A4), a conference room (A5), a tea room (A6), a dressing room (A7), a gymnasium, a concert hall, a theater, an exhibition space, a hospital space, an airport space, a station space and a combination thereof, and the cloud computing service device (B) receives and compares at least two or more of the air pollution data detected by the plurality of gas detectors (3) in the indoor field unit (A), intelligently calculates to position the location of the air pollution in the indoor field unit (A), and intelligently selects to issue the control command to the air guiding device (1), wherein the air guiding device (1) closest to the location of the air pollution is enabled for the activation operation firstly, then other air guiding devices (1) are enabled for the activation operation, and the directional airflow is generated to guide the air pollution to the filter screen (2) for filtering and removal, thereby the air pollution in the indoor field unit (A) is cleaned quickly to reach the gas state of complete purification.

7. The indoor air pollution prevention system according to claim 1, wherein the indoor field unit (A) is a kitchen field unit (A8), and the cloud computing service device (B) receives and compares the air pollution data detected by the gas detector (3) in the indoor field unit (A), wherein when the air pollution data exceeds a safety detection value, the control command is intelligently selected and issued to the air guiding device (1) for the activation operation, and the air pollution is quickly guided to the filter screen (2) for filtering and removal, so that the air pollution in the indoor field unit (A) is cleaned to reach the gas state of complete purification.

8. The indoor air pollution prevention system according to claim 1, wherein the gas detector (3) is disposed on the air guiding device (1).

9. The indoor air pollution prevention system according to claim 1, wherein the indoor field unit (A) is a bathroom field unit (A9), and the cloud computing service device (B) receives and compares the air pollution data detected by the gas detector (3) in the indoor field unit (A), wherein when the air pollution data exceeds a safety detection value, the control command is intelligently selected and issued to the air guiding device (1) for the activation operation, and the air pollution is quickly guided to the filter screen (2) for filtering and removal, so that the air pollution in the indoor field unit (A) is cleaned to reach the gas state of complete purification, and the temperature and the humidity of the indoor field unit (A) are controlled.

10. The indoor air pollution prevention system according to claim 1, wherein the gas detector (3) comprises a controlling circuit board (31), a gas detection main part (32), a microprocessor (33) and a communicator (34), and the gas detection main part (32), the microprocessor (33) and the communicator (34) are integrally packaged on the controlling circuit board (31) and electrically connected to the controlling circuit board (31), wherein the microprocessor (33) controls the detection of the gas detection main part (32), the gas detection main part (32) detects the air pollution and outputs the gas detection data, and the microprocessor (33) processes and provides the gas detection data to the communicator (34) for an external communication transmission.

11. The indoor air pollution prevention system according to claim 10, wherein the gas detection main part (32) comprises:
a base (321) comprising:
a first surface (3211);
a second surface (3212) opposite to the first surface (3211);
a laser loading region (3213) hollowed out from the first surface (3211) to the second surface (3212);
a gas-inlet groove (3214) concavely formed from the second surface (3212) and disposed adjacent to the laser loading region (3213), wherein the gas-inlet groove (3214) comprises a gas-inlet (3214a) and two lateral walls, the gas-inlet (3214a) is in communication with an environment outside the base (321), and a transparent window (3214b) is opened on the two lateral walls and is in communication with the laser loading region (3213);
a gas-guiding-component loading region (3215) concavely formed from the second surface (3212) and in communication with the gas-inlet groove (2314), wherein a ventilation hole (3215a) penetrates a bottom surface of the gas-guiding-component loading region (3215); and
a gas-outlet groove (3216) concavely formed from the first surface, (3211) spatially corresponding to the bottom surface of the gas-guiding-component loading region (3215), and hollowed out from the first surface (3211) to the second surface (3212) in a region where the first surface (3211) is not aligned with the gas-guiding-component loading region (3215), wherein the gas-outlet groove (3216) is in communication with the ventilation hole (3215a), and a gas-outlet (3216a) is disposed in the gas-outlet groove (3216);
a piezoelectric actuator (322) accommodated in the gas-guiding-component loading region (3215);
a driving circuit board (323) covering and attached to the second surface (3212) of the base (321);
a laser component (324) positioned and disposed on the driving circuit board (323), electrically connected to the driving circuit board (323), and accommodated in the laser loading region (3213), wherein a light beam path emitted from the laser component (324) passes through the transparent window (3214b) and extends in a direction perpendicular to the gas-inlet groove (3214), thereby forming an orthogonal direction with the gas-inlet groove (3214);
a particulate sensor (325) positioned and disposed on the driving circuit board (323), electrically connected to the driving circuit board (323), and disposed at an orthogonal position where the gas-inlet groove (3214) intersects the light beam path of the laser component (324) in the orthogonal direction, so that suspended particles contained in the air pollution passing through the gas-inlet groove (3214) and irradiated by a projecting light beam emitted from the laser component (324) are detected;
a gas sensor (327) positioned and disposed on the driving circuit board (323), electrically connected to the driving circuit board (323), and accommodated in the gas-outlet groove (3216), so as to detect the air pollution introduced into the gas-outlet groove (3216); and
an outer cover (326) covering the base (321) and comprising a side plate (3261), wherein the side plate (3261) has an inlet opening (3261a) and an outlet opening (3261b), the inlet opening (3261a) is spatially corresponding to the gas-inlet (3214a) of the base (321), and the outlet opening (3261b) is spatially corresponding to the gas-outlet (3216a) of the base (321);
wherein the outer cover (326) covers the base (321), and the driving circuit board (323) covers the second surface (3212), thereby an inlet path is defined by the gas-inlet groove (3214), and an outlet path is defined by the gas-outlet groove (3216), so that the air pollution is inhaled from the environment outside the base (321) by the piezoelectric actuator (322), transported into the inlet path defined by the gas-inlet groove (3214) through the inlet opening (3261a), and passes through the particulate sensor (325) to detect the particle concentration of the suspended particles contained in the air pollution, and the air pollution transported through the piezoelectric actuator (322) is transported out of the outlet path defined by the gas-outlet groove (3216) through the ventilation hole (3215a), passes through the gas sensor (327) for detecting, and then discharged through the outlet opening (3261b).

12. The indoor air pollution prevention system according to claim 11, wherein the particulate sensor (325) is used for detecting suspended particulate information.

13. The indoor air pollution prevention system according to claim 1, wherein the gas sensor comprises a formaldehyde sensor, a bacteria sensor, a virus sensor, a volatile-organic-compound sensor or a combination thereof, the formaldehyde sensor is used for detecting gas information of formaldehyde (HCHO), the bacteria sensor is used for detecting gas information of bacteria or fungi, the virus sensor used for detecting gas information of virus, and the volatile-organic-compound sensor is used for detecting gas information of carbon dioxide (CO₂) or volatile organic compounds (TVOC).

14. The indoor air pollution prevention system according to claim 1, wherein the filter screen (2) is a high efficiency particulate air (HEPA) filter screen (2a) to clean the air pollution through a physical way of blocking and absorbing, and the high efficiency particulate air (HEPA) filter screen (2a) is combined with a decomposition layer (21) to clean the air pollution through a chemical way, wherein the decomposition layer (21) comprises at least one selected from the group consisting of an activated carbon (21a), a cleansing factor containing chlorine dioxide layer (21b), an herbal protective layer (21c) extracted from ginkgo and Japanese rhus chinensis, a silver ion (21d), a zeolite (21e) and a combination thereof.

15. The indoor air pollution prevention system according to claim 1, wherein the filter screen (2) is combined with one selected form the group consisting of a light irradiation element (22), a decomposition unit (23) and a combination thereof to sterilize the air pollution in chemical means, wherein the light irradiation element (22) is at least one selected from the group consisting of a photo-catalyst unit comprising a photo catalyst (22a) and an ultraviolet lamp (22b), a photo-plasma unit comprising a nanometer irradiation tube (22c) and a combination thereof, wherein the decomposition unit (23) is at least one selected from the group consisting of a negative ion unit (23a), a plasma ion unit (23b) and a combination thereof.

## Patentansprüche

1. System zur Verhinderung von Innenraum-Luftverschmutzung, umfassend:
mindestens eine Innenraum-Feldeinheit (A), wobei die Innenraum-Feldeinheit (A) ein Raum ist, der durch eine Mehrzahl von Partitionen umgeben und isoliert ist, und eine Mehrzahl von Gasdetektoren (3), mindestens ein Filtersieb (2) und mindestens eine Luftleitvorrichtung (1) innerhalb der Innenraum-Feldeinheit (A) angeordnet sind, wobei jeder der Mehrzahl von Gasdetektoren (3) eine Charakteristik, eine Konzentration und einen Ort einer Luftverschmutzung erfasst und ausgibt, um Luftverschmutzungsdaten zur Verfügung zu stellen, das Filtersieb (2) die Luftverschmutzung in der durch dieses strömenden Luft filtert, und die Luftleitvorrichtung (1) die Luftverschmutzung leitet, um das Filtersieb (2) zwecks Filtern und Entfernen zu durchströmen;
mindestens eine Außenraum-Feldeinheit (C) mit mindestens einem darin angeordneten Gasdetektor (3); und
außerdem **gekennzeichnet durch** eine Cloud-Computing-Service-Vorrichtung (B), die ausgestaltet ist zum:
Empfangen der in der Innenraum-Feldeinheit (A) und in der Außenraum-Feldeinheit (C) erfassten Luftverschmutzungsdaten, Speichern der Luftverschmutzungsdaten in einer Luftverschmutzungsdatenbank, Implementieren einer Berechnung mit Hilfe künstlicher Intelligenz, um den Ort der Luftverschmutzung zu bestimmen, Ausgeben eines Steuerbefehls an die Luftleitvorrichtung, um eine Aktivierungsoperation der Luftleitvorrichtung (1) zu steuern, die dem Ort der Luftverschmutzung am nächsten ist, und anschließendes Steuern der Aktivierungsoperationen anderer Luftleitvorrichtungen (a), wodurch ein gerichteter Luftstrom erzeugt und die Luft, die die Luftverschmutzung enthält, schnell zum Filtersieb (2) geleitet wird, zwecks Filtern und Entfernen, um einen Gaszustand vollständiger Reinigung zu erreichen.

2. System zur Verhinderung von Innenraum-Luftverschmutzung nach Anspruch 1, wobei die Luftverschmutzung mindestens eine ist, die aus der Gruppe ausgewählt ist, die Partikel, Kohlenmonoxid, Kohlendioxid, Ozon, Schwefeldioxid, Stickstoffdioxid, Blei, die Summe aller flüchtigen organischen Verbindungen (TVOC), Formaldehyd, Bakterien, Pilze, Viren und eine Kombination davon enthält.

3. System zur Verhinderung von Innenraum-Luftverschmutzung nach Anspruch 1, wobei die Cloud-Computing-Service-Vorrichtung (B) ein Drahtlos-Netzwerk-Cloud-Computing-Service-Modul (B1), eine Cloud-Control-Service-Einheit (B2), eine Vorrichtungs-Management-Einheit (B3) und eine Anwendungsprogramm-Einheit (B4) umfasst.

4. System zur Verhinderung von Innenraum-Luftverschmutzung nach Anspruch 1, wobei die Innenraum-Feldeinheit (A) eine ist, die aus der Gruppe ausgewählt ist, die ein Wohnzimmer (A1), ein Schlafzimmer (A2), ein Familienzimmer (A3), ein Büro (A4), einen Konferenzraum (A5), ein Tee-Zimmer (A6), ein Ankleidezimmer (A7), einen Fitnessraum, einen Konzertsaal, ein Theater, einen Ausstellungsraum, einen Krankenhausraum, einen Flughafenraum, einen Bahnhofraum und eine Kombination davon enthält ist, und die Cloud-Computing-Service-Vorrichtung (B) die von den Mehrzahl von Gasdetektoren (3) in der Innenraum-Feldeinheit (A) und in der Außenraum-Feldeinheit (C) erfassten Luftverschmutzungsdaten empfängt und vergleicht, wobei, wenn die in der Innenraum-Feldeinheit (A) erfassten Luftverschmutzungsdaten höher sind als die in der Außenraum-Feldeinheit (C) erfassten Luftverschmutzungsdaten, die Cloud-Computing-Service-Vorrichtung (B) den Steuerbefehl für die Aktivierungsoperation an die Luftleitvorrichtung (1) ausgibt, die Luft in der Außenraum-Feldeinheit (C) zum Filtern durch das Filtersieb (2) geleitet wird und in die Innenraum-Feldeinheit (A) gelangt, und die Luftverschmutzung in der Innenraum-Feldeinheit (A) zum Filtersieb (2), zwecks Filtern und Entfernen, und zur Außenfeld-Einheit (C) geleitet wird, wodurch die Luft in der Innenfeld-Einheit (A) ausgetauscht wird, um einen Gaszustand vollständiger Reinigung zu erreichen.

5. System zur Verhinderung von Innenraum-Luftverschmutzung nach Anspruch 1, wobei das Filtersieb (2) in der Luftleitvorrichtung (1) angeordnet ist, um die Luftverschmutzung zu filtern, und die Luftleitvorrichtung (1) eine ist, die aus der Gruppe ausgewählt ist, die einen Frischluftventilator (11), einen Luftreiniger, einen Zirkulationsventilatorreiniger, einen Ventilator (12), ein Dunstabzugshaube (13), einen Abluftventilator (14) und eine Kombination davon enthält.

6. System zur Verhinderung von Innenraum-Luftverschmutzung nach Anspruch 1, wobei die Innenraum-Feldeinheit (A) eine ist, die aus der Gruppe ausgewählt ist, die ein Wohnzimmer (A1), ein Schlafzimmer (A2), ein Familienzimmer (A3), ein Büro (A4), einen Konferenzraum (A5), ein Tee-Zimmer (A6), ein Ankleidezimmer (A7), einen Fitnessraum, einen Konzertsaal, ein Theater, einen Ausstellungsraum, einen Krankenhausraum, einen Flughafenraum, einen Bahnhofsraum und eine Kombination davon enthält, und die Cloud-Computing-Service-Vorrichtung (B) mindestens zwei oder mehr der von der Mehrzahl von Gasdetektoren (3) in der Innenfeld-Einheit (A) erfassten Luftverschmutzungsdaten empfängt und vergleicht, auf intelligente Weise berechnet, um den Ort der Luftverschmutzung in der Innenraum-Feldeinheit (A) zu lokalisieren, und auf intelligente Weise auswählt, um den Steuerbefehl an die Luftleitvorrichtung (1) auszugeben, wobei die Luftleitvorrichtung (1), die dem Ort der Luftverschmutzung am nächsten ist, zuerst für die Aktivierungsoperation aktiviert wird, anschließend andere Luftleitvorrichtungen (1) für die Aktivierungsoperation aktiviert werden, und der gerichtete Luftstrom erzeugt wird, um die Luftverschmutzung zum Filtersieb (2) zu leiten, zwecks Filtern und Entfernen, wodurch die Luftverschmutzung in der Innenraum-Feldeinheit (A) schnell gereinigt wird, um einen Gaszustand vollständiger Reinigung zu erreichen.

7. System zur Verhinderung von Innenraum-Luftverschmutzung nach Anspruch 1, wobei die Innenraum-Feldeinheit (A) eine Küchen-Feldeinheit (A8) ist, und die Cloud-Computing-Service-Vorrichtung (B) die von dem Gasdetektor (3) in der Innenraum-Feldeinheit (A) erfassten Luftverschmutzungsdaten empfängt und vergleicht, wobei, wenn die Luftverschmutzungsdaten einen Sicherheitserfassungswert überschreiten, der Steuerbefehl intelligent ausgewählt und an die Luftleitvorrichtung (1) für die Aktivierungsoperation ausgegeben wird, und die Luftverschmutzung schnell zum Filtersieb (2) geleitet wird, zwecks Filtern und Entfernen, so dass die Luftverschmutzung in der Innenraum-Feldeinheit (A) gereinigt wird, um einen Gaszustand vollständiger Reinigung zu erreichen.

8. System zur Verhinderung von Innenraum-Luftverschmutzung nach Anspruch 1, wobei der Gasdetektor (3) an der Luftleitvorrichtung (1) vorgesehen ist.

9. System zur Verhinderung von Innenraum-Luftverschmutzung nach Anspruch 1, wobei die Innenraum-Feldeinheit (A) eine Badezimmer-Feldeinheit (A9) ist, und die Cloud-Computing-Service-Vorrichtung (B) die von dem Gasdetektor (3) in der Innenraum-Feldeinheit (A) erfassten Luftverschmutzungsdaten empfängt und vergleicht, wobei, wenn die Luftverschmutzungsdaten einen Sicherheitserfassungswert überschreiten, der Steuerbefehl intelligent ausgewählt und an die Luftleitvorrichtung (1) für die Aktivierungsoperation ausgegeben wird, und die Luftverschmutzung schnell zum Filtersieb (2) geleitet wird, zwecks Filtern und Entfernen, so dass die Luftverschmutzung in der Innenraum-Feldeinheit (A) gereinigt wird, um einen Gaszustand vollständiger Reinigung zu erreichen, und die Temperatur und die Feuchtigkeit der Innenraum-Feldeinheit (A) gesteuert werden.

10. System zur Verhinderung von Innenraum-Luftverschmutzung nach Anspruch 1, wobei der Gasdetektor (3) eine Steuerungsschaltungsplatine (31), einen Gasdetektionshauptteil (32), einen Mikroprozessor (33) und einen Kommunikator (34) umfasst, und der Gasdetektionshauptteil (32), der Mikroprozessor (33) und der Kommunikator (34) integriert auf der Steuerungsschaltungsplatine (31) vorgesehen und elektrisch mit der Steuerungsschaltungsplatine (31) verbunden sind, wobei der Mikroprozessor (33) die Detektion des Gasdetektionshauptteils (32) steuert, der Gasdetektionshauptteil (32) die Luftverschmutzung erfasst und die Gasdetektionsdaten ausgibt, und der Mikroprozessor (33) die Gasdetektionsdaten verarbeitet und dem Kommunikator (34) für eine externe Kommunikationsübertragung zur Verfügung stellt.

11. System zur Verhinderung von Innenraum-Luftverschmutzung nach Anspruch 10, wobei der Gasdetektionshauptteil (32) umfasst:
eine Basis (321), umfassend:
eine erste Fläche (3211);
eine zweite Fläche (3212), die der ersten Fläche (3211) gegenüberliegt;
eine Laserladeregion (3213), die von der ersten Fläche (3211) zur zweiten Fläche (3212) ausgehöhlt ist;
eine Gaseinlassnut (3214), die von der zweiten Fläche (3212) konkav ausgebildet und benachbart zur Laserladeregion (3213) vorgesehen ist, wobei die Gaseinlassnut (3214) einen Gaseinlass (3214a) und zwei Seitenwände aufweist, der Gaseinlass (3214a) mit einer Umgebung außerhalb der Basis (321) in Verbindung steht, und ein transparentes Fenster (3214b) an den beiden Seitenwänden geöffnet ist und mit der Laserladeregion (3213) in Verbindung steht;
eine Gasführungskomponentenladeregion (3215), die von der zweiten Fläche (3212) konkav gebildet ist und mit der Gaseinlassnut (2314) in Verbindung steht, wobei ein Belüftungsloch (3215a) eine Bodenfläche der Gasführungskomponentenladeregion (3215) durchdringt; und
eine Gasauslassnut (3216), die von der ersten Fläche (3211) konkav ausgebildet ist, räumlich der Bodenfläche der Gasführungskomponentenladeregion (3215) entspricht, und von der ersten Fläche (3211) zur zweiten Fläche (3212) in einer Region ausgehöhlt ist, wo die erste Fläche (3211) nicht mit der Gasführungskomponentenladeregion (3215) ausgerichtet ist, wobei die Gasauslassnut (3216) mit dem Belüftungsloch (3215a) in Verbindung steht, und ein Gasauslass (3216a) in der Gasauslassnut (3216) vorgesehen ist;
einen piezoelektrischen Aktuator (322), der in der Gasführungskomponentenladeregion (3215) untergebracht ist;
eine Ansteuerungsschaltungsplatine (323), die die zweite Fläche (3212) der Basis (321) bedeckt und an dieser befestigt ist;
eine Laserkomponente (324), die an der Ansteuerungsschaltungsplatine (323) positioniert und angeordnet ist, elektrisch mit der Ansteuerungsschaltungsplatine (323) verbunden ist, und in der Laserladeregion (3213) untergebracht ist, wobei ein von der Laserkomponente (324) emittierter Lichtstrahlpfad durch das transparente Fenster (3214b) hindurchgeht und sich in eine Richtung senkrecht zur Gaseinlassnut (3214) erstreckt, wodurch eine orthogonale Richtung mit der Gaseinlassnut (3214) gebildet wird;
einen Partikelsensor (325), der an der Ansteuerungsschaltungsplatine (323) positioniert und angeordnet ist, elektrisch mit der Ansteuerungsschaltungsplatine (323) verbunden ist, und an einer orthogonalen Position angeordnet ist, wo die Gaseinlassnut (3214) den Lichtstrahlpfad der Laserkomponente (324) in der orthogonalen Richtung kreuzt, so dass Schwebepartikel, die in der Luftverschmutzung enthalten sind, durch die Gaseinlassnut (3214) strömen und von einem projizierten Lichtstrahl, der von der Laserkomponente (324) emittierten wird, bestrahlt werden, erfasst werden;
einen Gassensor (327), der an der Ansteuerungssteuerungsplatine (323) positioniert und angeordnet ist, elektrisch mit der Ansteuerungsschaltungsplatine (323) verbunden ist, und in der Gasauslassnut (3216) untergebracht ist, um die Luftverschmutzung zu erfassen, die in die Gasauslassnut (3216) eingeleitet wird; und
eine äußere Abdeckung (326), die die Basis (321) abdeckt und eine Seitenplatte (3261) aufweist, wobei die Seitenplatte (3261) eine Einlassöffnung (3261a) und eine Auslassöffnung (3261b) aufweist, die Einlassöffnung (3261a) räumlich dem Gaseinlass (3214a) der Basis (321) entspricht, und die Auslassöffnung (3261b) räumlich dem Gasauslass (3216a) der Basis (321) entspricht;
wobei die äußere Abdeckung (326) die Basis (321) abdeckt, und die Ansteuerungsschaltungsplatine (323) die zweite Fläche (3212) abdeckt, wodurch ein Einlasspfad durch die Gaseinlassnut (3214) definiert wird, und ein Auslasspfad durch die Gasauslassnut (3216) definiert wird, so dass die Luftverschmutzung von der Umgebung außerhalb der Basis (321) durch den piezoelektrischen Aktuator (322) angesaugt, durch die Einlassöffnung (3261a) in den durch die Gaseinlassnut (3214) definierten Einlasspfad transportiert, und durch den Partikelsensor (325) geleitet wird, um die Partikelkonzentration der in der Luftverschmutzung enthaltenen Schwebepartikel zu erfassen, und die durch den piezoelektrischen Aktuator (322) transportierte Luftverschmutzung durch das Belüftungsloch (3215a) aus dem durch die Gasauslassnut (3216) definierten Auslasspfad transportiert wird, zwecks Detektion den Gassensor (327) durchströmt und dann durch die Auslassöffnung (3261b) ausgestoßen wird.

12. System zur Verhinderung von Innenraum-Luftverschmutzung nach Anspruch 11, wobei der Partikelsensor (325) zum Erfassen von Schwebepartikel-Informationen verwendet wird.

13. System zur Verhinderung von Innenraum-Luftverschmutzung nach Anspruch 1, wobei der Gassensor einen Formaldehyd-Sensor, einen Bakterien-Sensor, einen Virus-Sensor, einen Sensor für flüchtige organische Verbindungen oder eine Kombination davon umfasst, der Formaldehyd-Sensor zur Detektion von Gasinformationen bezüglich Formaldehyd (HCHO) verwendet wird, der Bakterien-Sensor zur Detektion von Gasinformationen bezüglich Bakterien oder Pilzen verwendet wird, der Virus-Sensor zur Detektion von Gasinformationen bezüglich Viren verwendet wird, und der Sensor für flüchtige organische Verbindungen zur Detektion von Gasinformationen bezüglich Kohlendioxid (CO₂) oder flüchtigen organischen Verbindungen (TVOC) verwendet wird.

14. System zur Verhinderung von Innenraum-Luftverschmutzung nach Anspruch 1, wobei das Filtersieb (2) ein HEPA-Filtersieb (2a) (High Efficiency Particulate Air Filtersieb) ist, um die Luftverschmutzung in physischer Weise durch Blockieren und Absorbieren zu reinigen, und das HEPA-Filtersieb (2a) mit einer Dekompositionsschicht (21) kombiniert ist, um die Luftverschmutzung in chemischer Weise zu reinigen, wobei die Dekompositionsschicht (21) mindestens eine enthält, die aus der Gruppe ausgewählt ist, die eine Aktivkohle (21a), eine Chlordioxid-Schicht (21b) mit Reinigungsfaktor, eine aus Ginkgo und japanischem Rhus Chinensis gewonnen pflanzliche Schutzschicht (21c), Silberionen (21d), Zeolith (21e) und eine Kombination davon enthält.

15. System zur Verhinderung von Innenraum-Luftverschmutzung nach Anspruch 1, wobei das Filtersieb (2) mit einem kombiniert ist, das aus der Gruppe ausgewählt ist, die ein Lichtbestrahlungselement (22), eine Dekompositionseinheit (23) und einer Kombination davon enthält, um die Luftverschmutzung auf chemische Weise zu sterilisieren, wobei das Lichtbestrahlungselement (22) mindestens eines ist, das aus der Gruppe ausgewählt ist, die eine Fotokatalysator-Einheit, die einen Fotokatalysator (22a) und eine Ultraviolett-Lampe (22b) aufweist, eine Fotoplasma-Einheit, die eine Nanometer-Bestrahlungsröhre (22c) aufweist, und eine Kombination davon enthält, wobei die Dekompositionseinheit (23) mindestens eine ist, die aus der Gruppe ausgewählt ist, die eine Negativionen-Einheit (23a), eine Plasmaionen-Einheit (23b) und eine Kombination davon enthält.

## Revendications

1. Système de prévention de pollution de l'air intérieur comprenant:
au moins une unité de champ intérieur (A), dans lequel l'unité de champ intérieur (A) est un espace entouré et isolé par une pluralité de cloisons, et une pluralité de détecteurs de gaz (3), au moins un tamis (2) et au moins un dispositif de guidage d'air (1) sont placés à l'intérieur de l'unité de champ intérieur (A), dans lequel chacun des détecteurs de gaz (3) détecte une caractéristique, une concentration et une localisation d'une pollution de l'air, et délivre en sortie des données de pollution de l'air, le tamis (2) filtre la pollution de l'air présente dans l'air qui le traverse, et le dispositif de guidage d'air (1) guide la pollution de l'air pour qu'elle passe à travers le tamis (2) et pour réaliser un filtrage et un retrait;
au moins une unité de champ extérieur (C) dans laquelle est placé au moins un détecteur de gaz (3); et
**caractérisé en ce qu'**il comprend en outre un dispositif de service informatique en nuage (B) configuré pour :
recevoir les données de pollution de l'air détectées dans l'unité de champ intérieur (A) et l'unité de champ extérieur (C), stocker les données de pollution de l'air dans une base de données de pollution de l'air, mettre en œuvre un calcul d'intelligence artificielle pour déterminer la localisation de la pollution de l'air, émettre une instruction de commande à destination du dispositif de guidage d'air pour commander une opération d'activation du dispositif de guidage d'air (1) au plus près de la localisation de la pollution de l'air, puis commander des opérations d'activation d'autres dispositifs de guidage d'air (a), moyennant quoi un flux d'air directionnel est produit, et l'air contenant la pollution de l'air est guidé rapidement vers le tamis (2) pour réaliser un filtrage et un retrait afin d'atteindre un état gazeux de purification complète.

2. Système de prévention de pollution de l'air intérieur selon la revendication 1, dans lequel la pollution de l'air est au moins une pollution choisie dans le groupe comprenant la matière particulaire, le monoxyde de carbone, le dioxyde de carbone, l'ozone, le dioxyde de soufre, le dioxyde d'azote, le plomb, les composés organiques volatils totaux (COVT), le formaldéhyde, les bactéries, les champignons, un virus et une combinaison de ceux-ci.

3. Système de prévention de pollution de l'air intérieur selon la revendication 1, dans lequel le dispositif de service informatique en nuage (B) comprend un module de service informatique en nuage de réseau sans fil (B1), une unité de service de commande de nuage (B2), une unité de gestion de dispositif (B3) et une unité de programme d'application (B4).

4. Système de prévention de pollution de l'air intérieur selon la revendication 1, dans lequel l'unité de champ intérieur (A) est une unité choisie dans le groupe comprenant un salon (A1), une chambre (A2), une chambre familiale (A3), un bureau (A4), une salle de réunion (A5), un salon de thé (A6), un vestiaire (A7), un gymnase, une salle de concert, un théâtre, un lieu d'exposition, un espace hospitalier, un espace d'aéroport, un espace de gare et une combinaison de ceux-ci, et le dispositif de service informatique en nuage (B) reçoit et compare les données de pollution de l'air détectées par la pluralité de détecteurs de gaz (3) dans l'unité de champ intérieur (A) et l'unité de champ extérieur (C), dans lequel si les données de pollution de l'air détectées dans l'unité de champ intérieur (A) sont supérieures aux données de pollution de l'air détectées dans l'unité de champ extérieur (C), le dispositif de service informatique en nuage (B) délivre l'instruction de commande au dispositif de guidage d'air (1) pour l'opération d'activation, l'air présent dans l'unité de champ extérieur (C) est introduit à travers le tamis (2) pour réaliser un filtrage et un retrait et entre dans l'unité de champ intérieur (A), et la pollution de l'air dans l'unité de champ intérieur (A) est guidée vers le tamis (2) pour réaliser un filtrage et un retrait vers l'unité de champ extérieur (C), moyennant quoi l'air présent dans l'unité de champ intérieur (A) est échangé afin d'atteindre l'état gazeux de purification complète.

5. Système de prévention de pollution de l'air intérieur selon la revendication 1, dans lequel le tamis (2) est placé dans le dispositif de guidage d'air (1) pour filtrer la pollution de l'air, et le dispositif de guidage d'air (1) est un élément choisi dans le groupe comprenant un ventilateur à air frais (11), un purificateur d'air, un purificateur de ventilateur brasseur d'air, un ventilateur (12), une hotte (13), un ventilateur d'extraction (14) et une combinaison de ceux-ci.

6. Système de prévention de pollution de l'air intérieur selon la revendication 1, dans lequel l'unité de champ intérieur (A) est une unité choisie dans le groupe comprenant un salon (A1), une chambre (A2), une chambre familiale (A3), un bureau (A4), une salle de réunion (A5), un salon de thé (A6), un vestiaire (A7), un gymnase, une salle de concert, un théâtre, un lieu d'exposition, un espace hospitalier, un espace d'aéroport, un espace de gare et une combinaison de ceux-ci, et le dispositif de service informatique en nuage (B) reçoit et compare au moins deux ou plus des données de pollution de l'air détectées par la pluralité de détecteurs de gaz (3) dans l'unité de champ intérieur (A), calcule de façon intelligente pour positionner la localisation de la pollution de l'air dans l'unité de champ intérieur (A), puis sélectionne de façon intelligente pour émettre l'instruction de commande vers le dispositif de guidage d'air (1), dans lequel le dispositif de guidage d'air (1) le plus proche de la localisation de la pollution de l'air est activé pour l'opération d'activation en premier, puis d'autres dispositifs de guidage d'air (1) sont activés pour l'opération d'activation, et le flux d'air directionnel est produit pour guider la pollution de l'air vers le tamis (2) pour réaliser un filtrage et un retrait, la pollution de l'air dans l'unité de champ intérieur (A) étant ainsi traitée rapidement pour atteindre l'état gazeux de purification complète.

7. Système de prévention de pollution de l'air intérieur selon la revendication 1, dans lequel l'unité de champ intérieur (A) est une unité de champ de cuisine (A8), et le dispositif de service informatique en nuage (B) reçoit et compare les données de pollution de l'air détectées par le détecteur de gaz (3) dans l'unité de champ intérieur (A), dans lequel, quand les données de pollution de l'air dépassent une valeur de détection de sécurité, l'instruction de commande est sélectionnée de façon intelligente et délivrée au dispositif de guidage d'air (1) pour l'opération d'activation, et la pollution de l'air est rapidement guidée vers le tamis (2) pour réaliser un filtrage et un retrait, de sorte que la pollution de l'air dans l'unité de champ intérieur (A) est traitée pour atteindre l'état gazeux de purification complète.

8. Système de prévention de pollution de l'air intérieur selon la revendication 1, dans lequel le détecteur de gaz (3) est placé sur le dispositif de guidage d'air (1).

9. Système de prévention de pollution de l'air intérieur selon la revendication 1, dans lequel l'unité de champ intérieur (A) est une unité de champ de salle de bains (A9), et le dispositif de service informatique en nuage (B) reçoit et compare les données de pollution de l'air détectées par le détecteur de gaz (3) dans l'unité de champ intérieur (A), dans lequel, quand les données de pollution de l'air dépassent une valeur de détection de sécurité, l'instruction de commande est sélectionnée de façon intelligente et délivrée au dispositif de guidage d'air (1) pour l'opération d'activation, et la pollution de l'air est rapidement guidée vers le tamis (2) pour réaliser un filtrage et un retrait, de sorte que la pollution de l'air dans l'unité de champ intérieur (A) est traitée pour atteindre l'état gazeux de purification complète, et la température et l'humidité de l'unité de champ intérieur (A) sont régulées.

10. Système de prévention de pollution de l'air intérieur selon la revendication 1, dans lequel le détecteur de gaz (3) comprend une carte de circuit de commande (31), une partie principale de détection de gaz (32), un microprocesseur (33) et un appareil de communication (34), et la partie principale de détection de gaz (32), le microprocesseur (33) et l'appareil de communication (34) sont intégralement incorporés sur la carte de circuit de commande (31) et connectés électriquement à la carte de circuit de commande (31), dans lequel le microprocesseur (33) commande la détection de la partie principale de détection de gaz (32), la partie principale de détection de gaz (32) détecte la pollution de l'air et délivre en sortie les données de détection de gaz, et le microprocesseur (33) traite et fournit les données de détection de gaz à l'appareil de communication (34) pour une transmission de communication externe.

11. Système de prévention de pollution de l'air intérieur selon la revendication 10, dans lequel la partie principale de détection de gaz (32) comprend:
une base (321) comprenant:
une première surface (3211);
une deuxième surface (3212) à l'opposé de la première surface (3211);
une région de chargement au laser (3213) évidée de la première surface (3211) à la deuxième surface (3212);
une rainure d'entrée de gaz (3214) formée de manière concave depuis la deuxième surface (3212) et adjacente à la région de chargement au laser (3213), dans laquelle la rainure d'entrée de gaz (3214) comprend une entrée de gaz (3214a) et deux parois latérales, l'entrée de gaz (3214a) étant en communication avec un environnement extérieur à la base (321), et une fenêtre transparente (3214b) étant ouverte sur les deux parois latérales et étant en communication avec la région de chargement au laser (3213);
une région de chargement de composant de guidage de gaz (3215) formée de manière concave depuis la deuxième surface (3212) et en communication avec la rainure d'entrée de gaz (3214), dans laquelle un trou de ventilation (3215a) traverse une surface inférieure de la région de chargement de composant de guidage de gaz (3215); et
une rainure de sortie de gaz (3216) formée de manière concave depuis la première surface (3211), en correspondance spatiale avec la surface inférieure de la région de chargement de composant de guidage de gaz (3215), et évidée de la première surface (3211) à la deuxième surface (3212) dans une région où la première surface (3211) n'est pas alignée avec la région de chargement de composant de guidage de gaz (3215), dans laquelle la rainure de sortie de gaz (3216) est en communication avec le trou de ventilation (3215a), et une sortie de gaz (3216a) est placée dans la rainure de sortie de gaz (3216);
un actionneur piézoélectrique (322) placé dans la région de chargement de composant de guidage de gaz (3215);
une carte de circuit de pilotage (323) couvrant la deuxième surface (3212) de la base (321) et fixée à celle-ci;
un composant laser (324) positionné et placé sur la carte de circuit de pilotage (323), connecté électriquement à la carte de circuit de pilotage (323), et placé dans la région de chargement au laser (3213), dans lequel un trajet de faisceau lumineux émis par le composant laser (324) passe à travers la fenêtre transparente (3214b) et s'étend dans une direction perpendiculaire à la rainure d'entrée de gaz (3214), formant ainsi une direction orthogonale avec la rainure d'entrée de gaz (3214);
un détecteur de particules (325) positionné et placé sur la carte de circuit de pilotage (323), connecté électriquement à la carte de circuit de pilotage (323), et disposé en une position orthogonale où la rainure d'entrée de gaz (3214) coupe le trajet de faisceau lumineux du composant laser (324) dans la direction orthogonale, de sorte que des particules suspendues contenues dans la pollution de l'air passant dans la rainure d'entrée de gaz (3214) et irradiées par un faisceau lumineux émis par le composant laser (324) sont détectées;
un détecteur de gaz (327) positionné et placé sur la carte de circuit de pilotage (323), connecté électriquement à la carte de circuit de pilotage (323), et placé dans la rainure de sortie de gaz (3216), afin de détecter la pollution de l'air introduite dans la rainure de sortie de gaz (3216); et
un couvercle extérieur (326) couvrant la base (321) et comprenant une plaque latérale (3261), dans lequel la plaque latérale (3261) comporte une ouverture d'entrée (3261a) et une ouverture de sortie (3261b), l'ouverture d'entrée (3261a) étant en correspondance spatiale avec l'entrée de gaz (3214a) de la base (321), et l'ouverture de sortie (3261b) étant en correspondance spatiale avec la sortie de gaz (3216a) de la base (321);
dans lequel le couvercle extérieur (326) couvre la base (321), et la carte de circuit de pilotage (323) couvre la deuxième surface (3212), un chemin d'entrée étant ainsi défini par la rainure d'entrée de gaz (3214), et un chemin de sortie étant défini par la rainure de sortie de gaz (3216), de sorte que la pollution de l'air est aspirée dans l'environnement extérieur de la base (321) par l'actionneur piézoélectrique (322), transportée dans le chemin d'entrée défini par la rainure d'entrée de gaz (3214) à traves l'ouverture d'entrée (3261a), et passe à travers le détecteur de particules (325) pour détecter la concentration en particules des particules suspendues contenues dans la pollution de l'air, et la pollution de l'air transportée à travers l'actionneur piézoélectrique (322) est transportée hors du chemin de sortie défini par la rainure de sortie de gaz (3216) à travers le trou de ventilation (3215a), passe dans le détecteur de gaz (327) pour réaliser une détection, puis est déchargée à travers l'ouverture de sortie (3261b).

12. Système de prévention de pollution de l'air intérieur selon la revendication 11, dans lequel le détecteur de particules (325) est utilisé pour détecter une information de particules suspendues.

13. Système de prévention de pollution de l'air intérieur selon la revendication 1, dans lequel le détecteur de gaz comprend un détecteur de formaldéhyde, un détecteur de bactéries, un détecteur de virus, un détecteur de composés organiques volatils ou une combinaison de ceux-ci, le détecteur de formaldéhyde est utilisé pour détecter une information de gaz de formaldéhyde (HCHO), le détecteur de bactéries est utilisé pour détecter une information de gaz de bactéries ou de champignons, le détecteur de virus est utilisé pour détecter une information de gaz de virus, et le détecteur de composés organiques volatils est utilisé pour détecter une information de gaz de dioxyde de carbone (CO₂) ou de composés organiques volatils (COVT).

14. Système de prévention de pollution de l'air intérieur selon la revendication 1, dans lequel le tamis (2) est un filtre à haute efficacité (HEPA) (2a) pour traiter la pollution de l'air par une méthode physique de blocage et d'absorption, et le filtre à haute efficacité (HEPA) (2a) est associé à une couche de décomposition (21) pour traiter la pollution de l'air par une méthode chimique, dans lequel la couche de décomposition (21) comprend au moins un élément choisi dans le groupe comprenant un charbon actif (21a), une couche de dioxyde de chlore contenant un facteur nettoyant (21b), une couche protectrice à base de plantes (21c) extraites du ginkgo et du rhus chinensis japonais, un ion argent (21d), une zéolite (21e) et une combinaison de ceux-ci.

15. Système de prévention de pollution de l'air intérieur selon la revendication 1, dans lequel le tamis (2) est associé à un élément choisi dans le groupe comprenant un élément émetteur de lumière (22), une unité de décomposition (23) et une combinaison de ceux-ci pour stériliser la pollution de l'air par voie chimique, dans lequel l'élément émetteur de lumière (22) est au moins un élément choisi dans le groupe comprenant une unité de photocatalyseur comprenant un photocatalyseur (22a) et une lampe à rayons ultraviolets (22b), une unité de photoplasma comprenant un tube de rayonnement nanométrique (22c) et une combinaison de ceux-ci, dans lequel l'unité de décomposition (23) est au moins une unité choisie dans le groupe comprenant une unité d'ion négatif (23a), une unité de plasma ionisé (23b) et une combinaison de celles-ci.
